# EUROPEAN PATENT SPECIFICATION

(11) **EP 3 383 835 B1**
(45) Date of publication and mention of the grant of the patent: **24.07.2019**
(21) Application number: 16815656.0
(22) Date of filing: 02.12.2016
(51) Int. Cl.: C07C 68/08, C07C 37/00, C07C 37/055, C07C 37/50, C07C 37/52, C07C 37/74, C08G 64/40, C07C 69/96, C07C 39/04

(54) **A METHOD FOR RECOVERING AN AROMATIC ALCOHOL FROM A MELT POLYMERIZATION, AND A SYSTEM FOR RECOVERING THE SAME**
VERFAHREN ZUR GEWINNUNG EINES AROMATISCHEN ALKOHOLS AUS EINER SCHMELZPOLYMERISATION UND SYSTEM ZUR RÜCKGEWINNUNG DAVON
PROCÉDÉ DE RÉCUPÉRATION D'UN ALCOOL AROMATIQUE À PARTIR D'UNE POLYMÉRISATION EN MASSE FONDUE ET SYSTÈME POUR SA RÉCUPÉRATION

(30) Priority: 03.12.2015 EP 15382605
(43) Date of publication of application: 10.10.2018
(73) Proprietor: SABIC Global Technologies B.V., 4612 PX Bergen op Zoom (NL)
(72) Inventor: FERNANDEZ, Ignacio Vic, 30390 La Aljorra (ES); AGUDO, Jorge A. Garcia, 30390 La Aljorra (ES); NADAL, Sergio Ferrer, 30390 La Aljorra (ES); BOJARSKI, Aaron David, 30390 La Aljorra (ES)
(74) Representative: Modiano, Micaela Nadia
(86) International application number: PCT/IB2016/057328
(87) International publication number: WO 2017/093974

(56) References cited:
- WO-A1-2006/049987
- WO-A1-2015/155737

## Description

### TECHNICAL FIELD

This disclosure relates to a method for recovering an aromatic alcohol from a melt polymerization, and especially to recovering aromatic alcohol and alkyl carbonates, and a system for recovering the same.

### BACKGROUND

Polycarbonate is a widely used raw material in many different manufacturing sectors. Due to the hardness and transparency of the material, it can be applied in applications as diverse as automotive windows and optical lenses. It is believed that the demand for polycarbonate will increase significantly in the coming years, requiring improvement in the production of polycarbonate, particularly in terms of efficiency and environmental impact. Polycarbonate can be melt polymerized via the reaction of a dihydroxy compound, such as a bisphenol, and a carbonate source, such as a diaryl carbonate.

WO2006/049987 discloses a method of making polycarbonates comprising melt-polymerizing an aromatic dihydroxy compound and a carbonic acid diester in the presence of a polymerization catalyst in a reactor system producing a byproduct stream, wherein the polymerization catalyst comprises a quaternary phosphonium compound; and purifying the by product stream to separate phenol, wherein the separated phenol has a phosphorus concentration of less than or equal to about 3 part per million, based upon the total weight of the phenol separated.

An improved method of melt polymerizing polycarbonate is desired.

### BRIEF SUMMARY

Disclosed herein is a method for recovering aromatic alcohol and/or alkyl carbonate from a melt polymerization, and a system for recovering the same.

In an embodiment, a method of purifying an aromatic alcohol comprises melt polymerizing a dihydroxy compound and a carbonate compound in the presence of a transesterification catalyst to form the aromatic alcohol and a polycarbonate in a melt polymerization facility; removing the aromatic alcohol in an overhead stream; separating the overhead stream in an aromatic alcohol purification unit into a purified aromatic alcohol stream comprising the aromatic alcohol and a purification unit bottom stream; separating the purification unit bottom stream in a diaryl carbonate purification unit into a diaryl carbonate stream and a diaryl carbonate purification unit bottom stream; and cracking a compound in the diaryl carbonate purification unit bottom stream in a cracker unit in the presence of a cracking agent to form a resultant stream comprising additional aromatic alcohol.

In another embodiment, a system for purifying an overhead stream from a melt polymerization facility, comprises an aromatic alcohol purification unit in fluid communication with the overhead stream from the melt polymerization facility, wherein the aromatic alcohol purification unit is capable of separating a purified aromatic alcohol stream and a purification unit bottom stream; a diary carbonate purification unit that is in fluid communication with the aromatic alcohol purification unit via the purification unit bottom stream, wherein diary carbonate purification unit is capable of separating a diaryl carbonate stream and a diaryl carbonate purification unit bottom stream; and a cracker unit that is in fluid communication with the diarylcarbonate purification unit via the diaryl carbonate purification unit bottom stream and a cracking agent stream, wherein the cracker unit is capable of forming a resultant stream comprising an additional aromatic alcohol.

The above described and other features are exemplified by the following figures and detailed description.

### BRIEF DESCRIPTION OF THE DRAWINGS

Refer now to the figures, which are exemplary embodiments, and wherein the like elements are numbered alike.
FIG. 1 is an illustration of a prior method for purifying an overhead stream from a melt polymerization facility;
FIG. 2 is an illustration of an embodiment of a method for purifying an overhead stream from a melt polymerization facility, e.g., enabling the recovery of aromatic alcohol and/or alkyl carbonate; and
FIG. 3 is an illustration of an embodiment of a section of a melt polymerization facility.

### DETAILED DESCRIPTION

Polycarbonate can be melt polymerized via the reaction of a dihydroxy compound and a diaryl carbonate to result in the polycarbonate and an aromatic alcohol side product. The aromatic alcohol can be removed via an overhead system, where, in early stages of the polymerization, for example, where a low vacuum is applied, the overhead stream can comprise greater than or equal to 99 weight percent (wt%) aromatic alcohol and only a minor amount of a monomer such as the diaryl carbonate. During later stages of the polymerization, the overhead stream can comprise as little as 50 wt% aromatic alcohol, as much as 40 wt% diaryl carbonate and as much as 10 wt% dihydroxy compound.

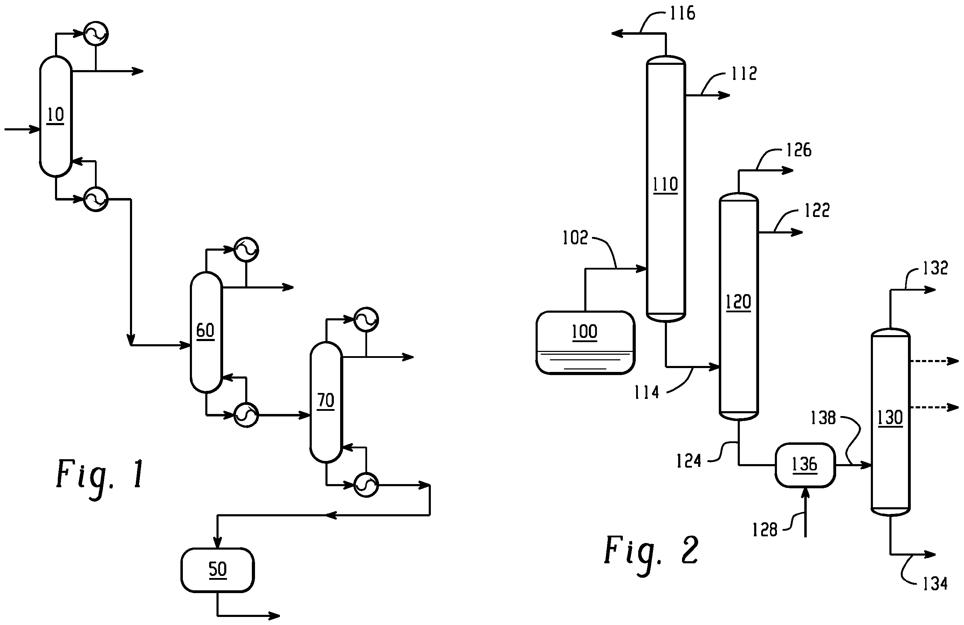

Methods of purification for purifying overhead streams from these later stages can rely on distillation column(s) to separate by-produced phenol from heavier boiling point compounds such as diphenyl carbonate. An example of such a purification unit is illustrated in FIG. 1. Here, an overhead stream from a melt polymerization facility is directed to a first phenol separation column 10 to separate a first separation top stream comprising purified phenol and a first separation bottom stream comprising diphenyl carbonate, bisphenol A, and oligomers. The first separation bottom stream is directed to phenyl methyl carbonate separation column 60 to separate a phenyl methyl carbonate top stream comprising a purified phenyl methyl carbonate (PMC) and a PMC column bottom stream. The PMC column bottom stream is directed to diphenyl carbonate separation column 70 to separate a diphenyl carbonate rich top stream and a separation column bottom stream comprising diphenyl carbonate, bisphenol A, oligomers, and catalyst. The separation column bottom stream is directed to furnace 50 for incineration.

Using such a scheme can disadvantageously result in a diphenyl carbonate stream comprising a high concentration of impurities (for example, diphenyl carbonate stream can have a maximum purity of only 99.7 wt% diphenyl carbonate) thus rendering the stream as unsuitable as a recycle stream for the melt polymerization. Such incorporation disadvantageously results in an increased amount of impurities in the diphenyl carbonate and can ultimately result in a reduced quality polycarbonate. The scheme can further disadvantageously result in the loss of any dihydroxy compound to ash.

Disclosed herein is a method of recovering one or more of the diaryl carbonate, the dihydroxy compound, and the aromatic alcohol and utilizing at least one of the dihydroxy compound and the aromatic alcohol in a monomer production facility. The method can result in one or more of an increased amount of aromatic alcohol (e.g., phenol) and/or alkyl carbonate being recovered from the melt polymerization overheads, an improved purity diaryl carbonate, and an improved quality melt polycarbonate.

The present process involves separating the one or more overhead streams from a melt polymerization in an aromatic alcohol purification unit into a purified aromatic alcohol stream comprising an aromatic alcohol and a purification unit bottom stream; separating the purification unit bottom stream in a diaryl carbonate purification unit into a diaryl carbonate stream and a diaryl carbonate purification unit bottom stream; and cracking the diaryl carbonate purification unit bottom stream in a cracker unit in the presence of a cracking agent to form an aromatic alcohol stream and a heavy boiler bottom stream. Prior to this disclosure, it was not believed that such a process would be energetically unfavorable, for example, due to the high cracking temperatures and pressures generally observed during the cracking process. It was surprisingly discovered that the present method, comprising cracking the diaryl carbonate purification unit bottom stream, could result in a further recovery of the aromatic alcohol and that the cracking could occur, for example, at a cracking temperature of 100 to 500 degrees Celsius (°C) (e.g., 150C to 400°C, or 200 to 400°C) and a pressure of 100 to 5,000 kilopascals (kPa) (e.g., 1,000 to 4000 kPa). The cracking step can recover an aromatic alcohol from a dihydroxy compound. For example, if the dihydroxy compound comprises bisphenol A, then phenol can be recovered.

"Polycarbonate" as used herein means a polymer or copolymer having repeating structural carbonate units of formula (1) 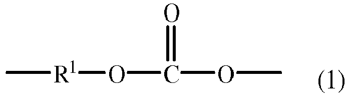 wherein at least 60% of the total number of R¹ groups are aromatic, or each R¹ contains at least one C₆₋₃₀ aromatic group. Specifically, each R¹ can be derived from a dihydroxy compound such as a dihydroxy compound of formula (2) or a dihydroxy compound of the bisphenol of formula (3). 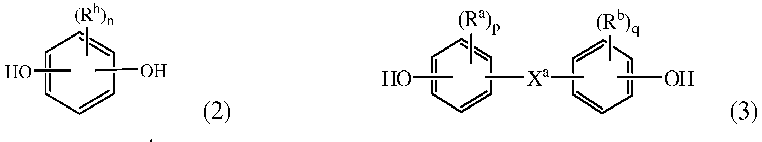 In formula (2), each R^{h} is independently a halogen atom, for example, bromine, a C₁₋₁₀ hydrocarbyl group such as a C₁₋₁₀ alkyl, a halogen-substituted C₁₋₁₀ alkyl, a C₆₋₁₀ aryl, or a halogen-substituted C₆₋₁₀ aryl, and n is 0 to 4.

In formula (3), R^{a} and R^{b} are each independently a halogen, C₁₋₁₂ alkoxy, or C₁₋₁₂ alkyl, and p and q are each independently integers of 0 to 4, such that when p or q is less than 4, the valence of each carbon of the ring is filled by hydrogen. In an embodiment, p and q are each 0, or p and q are each 1, and R^{a} and R^{b} are each a C₁₋₃ alkyl group, specifically methyl, disposed meta to the hydroxy group on each arylene group. X^{a} is a bridging group connecting the two hydroxy-substituted aromatic groups, where the bridging group and the hydroxy substituent of each C₆ arylene group are disposed ortho, meta, or para (specifically, para) to each other on the C₆ arylene group, for example, a single bond, -O-, -S-, -S(O)-, - S(O)₂-, -C(O)-, or a C₁₋₁₈ organic group, which can be cyclic or acyclic, aromatic or nonaromatic, and can further comprise heteroatoms such as halogens, oxygen, nitrogen, sulfur, silicon, or phosphorous. For example, X^{a} can be a substituted or unsubstituted C₃₋₁₈ cycloalkylidene; a C₁₋₂₅ alkylidene of the formula -C(R^{c})(R^{d})- wherein R^{c} and R^{d} are each independently hydrogen, C₁₋₁₂ alkyl, C₁₋₁₂ cycloalkyl, C₇₋₁₂ arylalkyl, C₁₋₁₂ heteroalkyl, or cyclic C₇₋₁₂ heteroarylalkyl; or a group of the formula -C(=R^{e})- wherein R^{e} is a divalent C₁₋₁₂ hydrocarbon group.

Some illustrative examples of dihydroxy compounds that can be used are described, for example, in WO 2013/175448 A1, US 2014/0295363, and WO 2014/072923.

The polycarbonate is prepared via a melt polymerization method and can be referred to herein as a melt polycarbonate. In the melt polymerization, the polycarbonate can be prepared by co-reacting, in a molten state, a dihydroxy compound and a carbonate compound in the presence of a transesterification catalyst. The reaction can be carried out in polymerization equipment, such as a continuously stirred reactor (CSTR), plug flow reactor, wire wetting fall polymerizers, free fall polymerizers, horizontal polymerizers, wiped film polymerizers, BANBURY mixers, single or twin screw extruders, or a combination comprising one or more of the foregoing. An aromatic alcohol is removed from the molten reactants in one or more overhead streams and the polymer is isolated as a molten residue. Melt polymerization can be conducted as a batch process or as a continuous process. In either case, the melt polymerization conditions used can comprise two or more distinct reaction stages.

For example, the polymerization can comprise an oligomerization stage, in which the starting dihydroxy compound and carbonate compound are converted into an oligomeric polycarbonate, and a second reaction stage also referred to as a polymerization stage wherein the oligomeric polycarbonate formed in the oligomerization stage is converted to high molecular weight polycarbonate. The oligomerization stage can comprise 1 or more, specifically, 2 to 4 oligomerization units (for example, 2 to 4 continuously stirred tanks). When 2 or more oligomerization units are present in a series, one or both of an increase in temperature and a decrease in pressure can occur from one unit to the next. The polymerization stage can comprise 1 or more, more specifically, 2 to 4 polymerization units (for example, 2 horizontal or wire wetting fall polymerizers). The polymerization stage can comprise one or more polymerization units that can polymerize the polycarbonate to a weight average molecular weight (Mw) of, for example, 25,000 to 140,000 Daltons based on polystyrene standards. Unless otherwise stated, molecular weights as used herein are determined using gel permeation chromatography based on polystyrene standards. After formation of a polycarbonate, the polycarbonate composition can then be optionally quenched and devolatilized in a devolatilization unit, where Mw of the polycarbonate does not significantly increase (for example, the molecular weight does not increase by greater than 10 wt%) and a temperature, a pressure, and a residence time are used to reduce the concentration of low molecular weight components.

The oligomerization unit is herein defined as a unit that results in polycarbonate oligomers with a Mw of less than or equal to 25,000 Daltons and a polymerization unit is herein defined as a unit that produces polycarbonate with a Mw of greater than 25,000 Daltons. It is noted that while less than or equal to 25,000 Daltons is used here to define a Mw achieved in the oligomerization stage, one skilled in the art readily understands that said Mw is used merely to define an oligomerization stage, where the oligomer Mw could be greater than 25,000 Daltons. A "staged" polymerization reaction condition can be used in continuous polymerization systems, wherein the starting monomers are oligomerized in a first reaction vessel and the oligomeric polycarbonate formed therein is continuously transferred to one or more downstream reactors in which the oligomeric polycarbonate is converted to a higher Mw polycarbonate. Typically, in the oligomerization stage, the oligomeric polycarbonate produced has an Mw of 1,000 to 25,000 Daltons. In one or more subsequent polymerization stages, the Mw of the polycarbonate can be increased to, for example, 25,000 to 140,000 Daltons (using polystyrene standard), specifically, 30,000 to 70,000 Daltons.

Typically, solvents are not used in the melt polymerization, and the reactants including the dihydroxy aromatic compound and the diaryl carbonate are in a molten state. The reaction temperature can be 100 to 350°C, specifically, 180 to 310°C. The pressure can be at atmospheric pressure, supra-atmospheric pressure, or a range of pressures from atmospheric pressure to 15 torr (2 kilopascal) in the initial stages of the reaction, and at a reduced pressure at later stages, for example, 0.2 to 15 torr (26 pascal to 2 kilopascal).

The polymerization can occur in a series of polymerization vessels that can each individually have increasing temperature and/or vacuum. For example, an oligomerization stage can occur at a temperature of 100 to 280°C, specifically, 140 to 240°C and a polymerization stage can occur at a temperature of 240 to 350°C, specifically, 280 to 300°C, or 240 to 270°C, or 250 to 310°C, where the temperature in the polymerization stage is greater than the temperature in the oligomerization stage. The oligomerization can occur at a pressure of greater than or equal to 10 kilopascals absolute (kPa(a)) or the oligomerization can comprise at least two oligomerization units where a first oligomerization unit can have a pressure of greater than or equal to 10 kPa(a) and a second oligomerization can have a pressure of 1.5 to 9 kPa(a), where the first oligomerization unit is upstream of the second oligomerization unit, where one or more oligomerization units can be located before the polymerization units.

The polymerization stage following the oligomerization stage can comprise polymerizing in one or multiple (e.g., two) polymerization units. The first polymerization unit can be at a temperature of 240 to 350°C, specifically, 260 to 310°C and a pressure of 0.1 to 1 kPa(a). The second polymerization unit can, for example, be at a temperature of 240 to 350°C, specifically, 260 to 300°C and a pressure of less than or equal to 0.5 kPa(a). The second polymerization unit can be operated at a temperature greater than the temperature of the first polymerization unit. The second polymerization unit can be operated at a pressure less than the pressure of the first polymerization unit. The polycarbonate can be devolatilized after a final polymerization.

The reaction time from the initial oligomerization unit to the final polymerization unit can be 0.1 to 15 hours. A final polymerization unit as used herein refers to a final polymerization unit in the melt polymerization where the last increase in molecular weight occurs. A quenching agent can be added to the polycarbonate resin after a final polymerization, and optionally, before any melt filtering.

After a final polymerization vessel (also referred to as a final polymerization unit), the polymer can be introduced to a reactor, extruded, subjected to filtration in a melt filter, or a combination comprising one or more of the foregoing. It is noted that the melt filter can be located before or after the extruder. For example, the melt polymerization process for the manufacture of a polycarbonate composition can comprise: melt polymerizing a dihydroxy reactant and a carbonate compound to produce a molten reaction product; quenching the molten reaction product; filtering the molten reaction product in a melt filter upstream of any extruders; optionally introducing an additive to form a mixture; and extruding the mixture to form the polycarbonate composition. Likewise, the melt polymerization process for the manufacture of a polycarbonate composition can comprise: melt polymerizing a polycarbonate; introducing a quencher composition and optionally an additive to form a mixture; and extruding the mixture to form the polycarbonate composition.

The transesterification catalysts used in the melt transesterification polymerization production of polycarbonates can include one or both of an alkali catalyst and a quaternary catalyst, wherein the alkali catalyst comprises a source of at least one of alkali ions and alkaline earth ions, and wherein the quaternary catalyst comprising a quaternary ammonium compound, a quaternary phosphonium compound, or a combination comprising at least one of the foregoing. The quaternary catalyst can have a reduced metal salt concentration.

The alkali catalyst comprises a source of one or both of alkali ions and alkaline earth ions. The sources of these ions include alkaline earth hydroxides such as magnesium hydroxide and calcium hydroxide. Sources of alkali metal ions can include the alkali metal hydroxides such as illustrated by lithium hydroxide, sodium hydroxide, potassium hydroxide, and combinations comprising at least one of the foregoing. Examples of alkaline earth metal hydroxides are calcium hydroxide, magnesium hydroxide, and combinations comprising at least one of the foregoing. The alkali catalyst can comprise sodium hydroxide. The alkali catalyst typically will be used in an amount sufficient to provide 1 x 10⁻² to 1 x 10⁻⁸ moles, specifically, 1 x 10⁻⁴ to 1 x 10⁻⁷ moles of metal hydroxide per mole of the dihydroxy compounds employed. Other possible sources of alkaline earth and alkali metal ions include salts of carboxylic acids (such as sodium acetate) and derivatives of ethylene diamine tetraacetic acid (EDTA) (such as EDTA tetrasodium salt, and EDTA magnesium disodium salt), as well as combinations comprising at least one of the foregoing. For example, the alkali catalyst can comprise alkali metal salt(s) of a carboxylic acid, alkaline earth metal salt(s) of a carboxylic acid, or a combination comprising at least one of the foregoing. The alkali catalyst can comprise Na₂Mg EDTA or a salt thereof.

The alkali can also, or alternatively, comprise salt(s) of a non-volatile inorganic acid. For example, the alkali catalyst can comprise salt(s) of a non-volatile inorganic acid such as NaH₂ PO₃, NaH₂PO₄, Na₂HPO₃, KH₂PO₄, CsH₂PO₄, Cs₂HPO₄, and combinations comprising at least one of the foregoing. Alternatively, or in addition, the alkali catalyst can comprise mixed alkali metal salt(s) of phosphoric acid, such as NaKHPO₄, CsNaHPO₄, CsKHPO₄, and combinations comprising at least one of the foregoing. The alkali catalyst can comprise KNaHPO₄, wherein a molar ratio of Na to K is 0.5 to 2.

The quaternary catalyst can comprise a quaternary ammonium compound, a quaternary phosphonium compound, or a combination comprising at least one of the foregoing. The quaternary ammonium compound can be an organic ammonium compound(s) having structure, (R⁴)₄N⁺X⁻, wherein each R⁴ is the same or different, and is a C₁₋₂₀ alkyl, a C₄₋₂₀ cycloalkyl, or a C₄₋₂₀ aryl; and X⁻ is an organic or inorganic anion, for example, a hydroxide, halide, carboxylate, sulfonate, sulfate, formate, carbonate, or bicarbonate. Some non-limiting examples of organic quaternary ammonium compounds include tetramethyl ammonium hydroxide, tetrabutyl ammonium hydroxide, tetramethyl ammonium acetate, tetramethyl ammonium formate, tetrabutyl ammonium acetate, and combinations comprising at least one of the foregoing. Tetramethyl ammonium hydroxide is often employed.

The quaternary phosphonium compound can be of organic phosphonium compounds having structure, (R⁵)₄P⁺X⁻, wherein each R⁵ is the same or different, and is a C₁₋₂₀ alkyl, a C₄₋₂₀ cycloalkyl, or a C₄₋₂₀ aryl; and X⁻ is an organic or inorganic anion, for example, a hydroxide, phenoxide, halide, carboxylate such as acetate or formate, sulfonate, sulfate, formate, carbonate, or bicarbonate. Where X⁻ is a polyvalent anion such as carbonate or sulfate, it is understood that the positive and negative charges in the quaternary ammonium and phosphonium structures are properly balanced. Where each R⁵ independently is a methyl group and X⁻ is carbonate, it is understood that X⁻ represents 2(CO₃⁻²).

Examples of quaternary phosphonium compounds include tetramethyl phosphonium hydroxide, tetramethyl phosphonium acetate, tetramethyl phosphonium formate, tetrabutyl phosphonium hydroxide, tetraphenyl phosphonium acetate (TPPA), tetraphenyl phosphonium phenoxide (TPPP), tetraethyl phosphonium acetate, tetrapropyl phosphonium acetate, tetrabutyl phosphonium acetate, tetrapentyl phosphonium acetate, tetrahexyl phosphonium acetate, tetraheptyl phosphonium acetate, tetraoctyl phosphonium acetate, tetradecyl phosphonium acetate, tetradodecyl phosphonium acetate, tetratolyl phosphonium acetate, tetramethyl phosphonium benzoate, tetraethyl phosphonium benzoate, tetrapropyl phosphonium benzoate, tetraphenyl phosphonium benzoate, tetraethyl phosphonium formate, tetrapropyl phosphonium formate, tetraphenyl phosphonium formate, tetramethyl phosphonium propionate, tetraethyl phosphonium propionate, tetrapropyl phosphonium propionate, tetramethyl phosphonium butyrate, tetraethyl phosphonium butyrate, and tetrapropyl phosphonium butyrate, and combinations comprising at least one of the foregoing. The quaternary catalyst can comprise TPPP, TPPA, or a combination comprising one or both of the foregoing.

The amount of second quaternary employed is typically based upon the total number of moles of dihydroxy compound employed in the polymerization reaction. When referring to the ratio of quaternary catalyst, for example, phosphonium salt, to all dihydroxy compounds employed in the polymerization reaction, it is convenient to refer to moles of phosphonium salt per mole of the dihydroxy compound(s), meaning the number of moles of phosphonium salt divided by the sum of the moles of each individual dihydroxy compound present in the reaction mixture. The amount of quaternary catalyst employed typically will be 1 x 10⁻² to 1 x 10⁻⁵, specifically, 1 x 10⁻³ to 1 x 10⁻⁴ moles per total mole of the dihydroxy compounds in the reaction mixture.

The quaternary catalyst can have a reduced concentration of metal compounds, e.g., the quaternary catalyst can comprise one or more of: a) less than or equal to 2,000 parts per million by weight (ppm), specifically, less than or equal to 1,675 ppm, specifically, less than or equal to 500 ppm, more specifically, less than or equal to 100 ppm, even more specifically, less than or equal to 30 ppm of sodium; b) less than or equal to 500 ppm, specifically, less than or equal to 300 ppm, more specifically, less than or equal to 135 ppm of cesium; and c) less than or equal to 100 ppm, specifically, less than or equal to 45 ppm of potassium; based on the total weight of the quaternary catalyst.

The quaternary catalyst can comprise an alkali metal compound, wherein if the compound comprises sodium sulfate, the amount of sodium can be less than or equal to 1,690 ppm, specifically, less than or equal to 1,670 ppm based on the total weight of the quaternary catalyst; if the compound comprises cesium sulfate, the amount of cesium can be less than or equal to 275 ppm, specifically, less than or equal to 252 ppm based on the total weight of the quaternary catalyst; if the compound comprises sodium hydroxide, the amount of sodium can be less than or equal to 35 ppm, specifically, less than or equal to 29 ppm based on the total weight of the quaternary catalyst; if the compound comprises potassium hydroxide, the amount of potassium can be less than or equal to 50 ppm, specifically, less than or equal to 43 ppm based on the total weight of the quaternary catalyst; if the compound comprises cesium hydroxide, the amount of cesium can be less than or equal to 140 ppm, specifically, less than or equal to 132 ppm based on the total weight of the quaternary catalyst; or a combination comprising one or more of the foregoing.

For example, the quaternary catalyst can comprise an alkali metal compound, wherein the amount of sodium can be greater than or equal to 1 ppm, or greater than or equal to 30 ppm, or greater than or equal to 100 ppm; the amount of cesium can be greater than or equal to 10 ppm, or greater than or equal to 30 ppm, or greater than or equal to 50 ppm; the amount of potassium can be greater than 0 ppm, or greater than or equal to 5 ppm, or greater than or equal to 10 ppm; or a combination comprising one or more of the foregoing, wherein the metal amounts are based on the weight of the quaternary catalyst.

A quencher composition can be added at one or more locations in the present melt preparation of the polycarbonate to reduce the activity of the catalyst. The quencher composition comprises a quenching agent (also referred to herein as a quencher). For example, the quenching agent can comprise a sulfonic acid ester such as an alkyl sulfonic ester of the formula R₁SO₃R₂ wherein R₁ is hydrogen, C₁-C₁₂ alkyl, C₆-C₁₈ aryl, or C₇-C₁₉ alkylaryl, and R₂ is C₁-C₁₂ alkyl, C₆-C₁₈ aryl, or C₇-C₁₉ alkylaryl. Examples of alkyl sulfonic esters include benzenesulfonate, p-toluenesulfonate, methylbenzene sulfonate, ethylbenzene sulfonate, n-butyl benzenesulfonate, octyl benzenesulfonate and phenyl benzenesulfonate, methyl p-toluenesulfonate, ethyl p-toluenesulfonate, n-butyl p-toluene sulfonate, octyl p-toluenesulfonate and phenyl p- toluenesulfonate. The sulfonic acid ester can comprise alkyl tosylates such as n-butyl tosylate. The sulfonic acid ester can be present in the quencher composition in an amount of 0.1 to 10 volume percent (vol%), specifically, 0.1 to 5 vol%, more specifically, 0.5 to 2 vol% based on the total volume of the quencher composition.

The quencher composition can be added to the polycarbonate at a pressure of greater than or equal to 2 bars and mixed with the polycarbonate for a period of time of greater than or equal to 5 seconds prior to the addition to the polycarbonate of any additives having a reactive OH group or reactive ester group. As used herein, when referring to "reactive" or a "reactive group", e.g., having a reactive OH⁻ group or a reactive ester group, the reactivity is with respect to polycarbonate.

The polycarbonate can be, for example, a bisphenol A polycarbonate with an Mw of 13,000 to 20,000 Daltons based on polystyrene standards.

The polycarbonate can have a melt flow of 4 to 40 g/10 min, for example, 4.5 to 15 g/10 min or 15 to 35 g/10 min as determined by ASTM D1238-04 at 300°C, 1.5 kg. The polycarbonate can have a melt flow of 5 to 15 g/10 min as determined by ASTM D1238-04 at 250°C, 1.5 kg.

The polycarbonates can have a low color value of, for example, a CIE b* index of less than or equal to 0.5, specifically, less than or equal to 0.15 as determined by spectrophotometry and high light transmission of, for example, greater than or equal to 89% as determined by spectrophotometry.

The polycarbonate can be further compounded, for example, to make a polycarbonate blend.

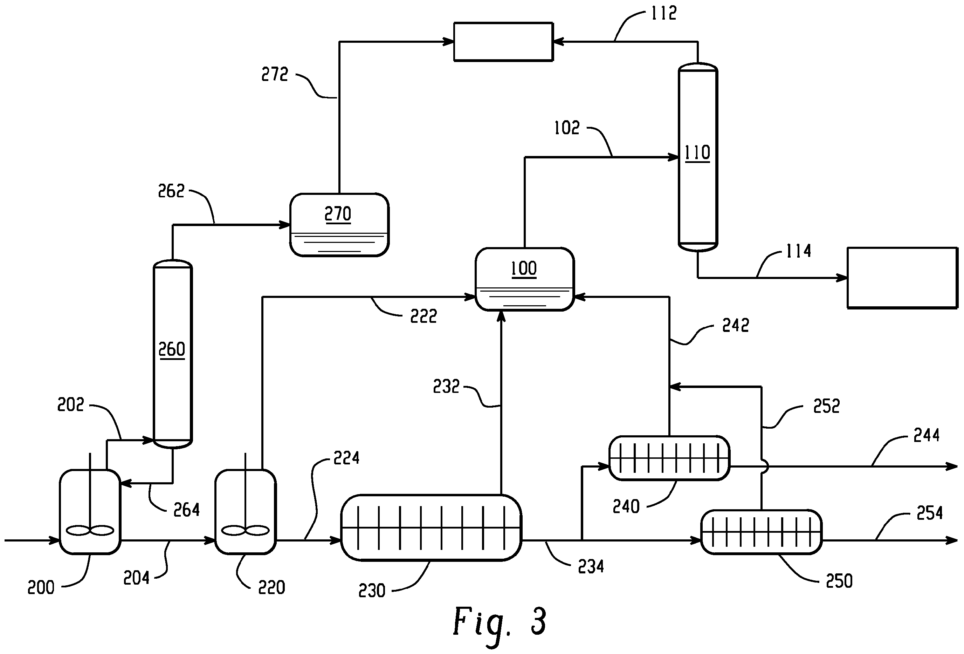

FIG. 3 illustrates a portion of a melt polymerization facility. Specifically, a first oligomerization unit 200 can react low molecular weight oligomers formed in a monomer mixing unit. The monomer mixing unit can react monomers present in a monomer mixture comprising a diaryl carbonate, a dihydroxy compound, and a catalyst. First oligomerization overhead stream 202 can be withdrawn from first oligomerization unit 200. First oligomerization overhead stream 202 can comprise greater than or equal to 95 wt%, specifically, 95 to 99 wt% aromatic alcohol based on the total weight of the first oligomerization overhead stream. First oligomerized stream 204 can be directed from first oligomerization unit 200 to second oligomerization unit 220. Second oligomerization overhead stream 222 can be withdrawn from second oligomerization unit 220. Second oligomerization overhead stream 222 can comprise 50 to 80 wt%, specifically, 70 to 80 wt% of aromatic alcohol based on the total weight of the second oligomerization overhead stream.

Second oligomerized stream 224 can be directed to first polymerization unit 230, from which first polymerization overhead stream 232 can be withdrawn. First polymerization overhead stream 232 can comprise 45 to 75 wt%, specifically, 65 to 75 wt% of the aromatic alcohol based on the total weight of the stream. The first polymerized stream 234 can be directed to a single final polymerization unit (e.g., primary final polymerization unit 240) or the stream 234 can be split into multiple streams and directed to more than one final polymerization unit (e.g., 240 and 250). The first polymerized stream 234 would be split if it was desired to produce different Mw polycarbonates from the first polymerized stream 234. When first polymerized stream 234 is directed to at least one of primary final polymerization unit 240 and secondary final polymerization unit 250, primary polymerization overhead stream 242 and secondary polymerization stream 252 can be withdrawn from the respective polymerization units. One or both of primary polymerization overhead stream 242 and secondary polymerization stream 252 can comprise 40 to 70 wt%, specifically, 55 to 70 wt% of the aromatic alcohol based on the total weight of the respective stream. Polycarbonate product streams 244 and 254 can each be directed to an extruder for pelletization.

First oligomerization overhead stream 202 can be directed to oligomerization overhead purification column 260. Bottom overhead stream 264 can be directed back to first oligomerization unit 200. Bottom overhead stream 264 can comprise 70 to 100 wt% of a diaryl carbonate based on the total weight of the stream. Top overhead stream 262 can be directed to a monomer production facility or to upstream tank 270. Top overhead stream 262 can comprise greater than or equal to 99 wt%, specifically, 99.5 to 100 wt%, more specifically, 99.7 to 100 wt% aromatic alcohol based on the total weight of the top overhead stream. Top overhead stream 262 can be in fluid communication with the monomer production facility, for example, via stream 272.

One or more of second oligomerization overhead stream 222, first polymerization overhead stream 232, primary polymerization overhead stream 242, and secondary polymerization overhead stream 252 can be directed to downstream tank 100 or can be directed directly to aromatic alcohol purification unit 110. When one or more of second oligomerization overhead stream 222, first polymerization overhead stream 232, primary polymerization overhead stream 242, and secondary polymerization overhead stream 252 are directed to downstream tank 100, then combined stream 102 can be directed to aromatic alcohol purification unit 110. Combined stream 102 can comprise one or more of 50 to 80 wt% of an aromatic alcohol; 15 to 35 wt% of a diaryl carbonate; 0.1 to 15 wt% of a dihydroxy compound; a catalyst; and a PC oligomer component (e.g., 0.1 to 10wt%); wherein the combined total of the components in the stream is 100 wt%.

Purified aromatic alcohol stream 112 can be removed as a top stream from aromatic alcohol purification unit 110, for example, as illustrated in FIG. 3. Conversely, purified aromatic alcohol stream 112 can be removed as a side stream from aromatic alcohol purification unit 110 and light boiler top stream 116 can be removed from the top of aromatic alcohol purification unit 110. Light boiler top stream 116 can comprise compounds that have a higher volatility than the aromatic alcohol. Purified aromatic alcohol stream 112 can comprise greater than or equal to 99 wt%, specifically, 99.5 to 100 wt%, more specifically, 99.7 to 100 wt% aromatic alcohol based on the total weight of the purified aromatic alcohol stream. Purified aromatic alcohol stream 112 can be directed to and can be in fluid communication with a monomer production facility.

Purification unit bottom stream 114 can be directed to diaryl carbonate purification unit 120. Diaryl carbonate stream 122 can be withdrawn from a top of diaryl carbonate purification unit 120 or as a side stream from diaryl carbonate purification unit 120. When withdrawn as a side stream, medium boiler top stream 126 can be withdrawn from a top of diaryl carbonate purification unit 120. Medium boiler top stream 126 can comprise compounds having a higher volatility than the diaryl carbonate and a lower volatility than the aromatic alcohol. Diaryl carbonate stream 122 can be recycled back to the melt polymerization facility or to the diaryl carbonate production unit. Diaryl carbonate purification unit bottom stream 124 can be directed from diaryl carbonate purification unit 120 to cracker unit 136 along with a cracking agent stream 128 (which can be added directly into the cracker unit 136, or mixed with stream 124 upstream of the cracker unit 136). Diaryl carbonate purification unit bottom stream 124 can comprise a dihydroxy compound, oligomers (e.g., PC oligomers), heavies (also referred to as high boilers; and wherein heavies include compounds that have a boiling point higher than the boiling point of DPC under the conditions of the unit they are located in), or a combination comprising at least one of the foregoing. Cracking agent stream 128 and diaryl carbonate purification unit bottom stream 124 can be added to cracker unit 136 as a single stream or as two separate streams.

FIG. 2 illustrates that cracking agent stream 128 can be added to cracking unit 136 that can further optionally function as a mixing unit. Cracking unit 136 can optionally be operated in a partial loop with heavy boiler bottom stream 134. In the partial loop at least a portion of heavy boiler bottom stream 134 can be directed back into optional unit 136. Separation unit stream 132, can be a single stream (e.g. a top stream), or can be multiple streams, e.g., a top stream and a side stream (e.g., 1, 2, or 3 side streams), wherein the side streams are illustrated in FIG. 2 as dashed, arrows from unit 130. Separation unit stream 132 can be directed to a monomer production facility. Optionally, the separation unit 130 can further be an additional cracking unit. Also, depending upon the desired level of separation, the separation unit 130 can be a multiple separation units.

Heavy boiler bottom stream 134 can be withdrawn from the bottom of cracker unit 130, for example, for disposal or use as a fuel. Heavy boiler bottom stream 134 can comprise materials with a lower volatility than para-para BPA, ortho-para BPA, or a combination comprising at least one of the foregoing.

The cracker unit can crack components such as dihydroxy compounds and PC oligomers into one or more of aromatic alcohols, dialkyl carbonates, and alkyl-aryl carbonates. The cracking can occur at a temperature of 100 to 500°C, specifically, 140 to 250°C. The cracking can occur at a pressure of 100 to 10,000 kPa, specifically, 100 to 5,000 kPa. The residence time in the cracker unit can be up to 20 hours, e.g., 0.01 to 10 hours, or 0.01 to 2 hours. The cracking can occur in an inert atmosphere, for example, in nitrogen. As used herein, the inert atmosphere is with respect to the cracking composition.

The cracking occurs in the presence of a cracking agent. The cracking agent can comprise water and/or alkyl alcohol. The alkyl alcohol can comprise; C₁₋₃₄ alkyl alcohol, specifically, C₁₋₆ alkyl alcohol, more specifically, C₁₋₄ alkyl alcohol. For example, the C₁₋₄ alkyl alcohol can comprise methanol, ethanol, n-propanol, isopropanol, n-butanol, sec-butanol, tert-butanol, or a combination comprising one or more of the foregoing. The C₁₋₆ alkanol can comprise n-pentanol, 1-methylbutanol, 2-methylbutanol, 3-methylbutanol, neopentanol, 1-ethylpropanol, cyclohexanol, cyclopentanol, n-hexanol, 1,1-dimethylpropanol, 1,2-dimethylpropanol, 1-methylpentanol, 2-methylpentanol, 3-methylpentanol, 4-methylpentanol, 1,1-dimethylbutanol, 1,2-dimethylbutanol, 1,3-dimethylbutanol, 2,2-dimethylbutanol, 2,3-dimethylbutanol, 3,3-dimethylbutanol, 1-ethylbutanol, 2-ethylbutanol, 1,1,2-trimethylpropanol, 1,2,2-trimethylpropanol, 1-ethyl-1-methylpropanol, 1-ethyl-2-methylpropanol, or a combination comprising one or more of the foregoing. The C₁-C₃₄-alkanol can comprise n-heptanol, n-octanol, pinacanol, adamantanol, an isomeric methanol, n-nonanol, n-decanol, n-dodecanol, n-tridecanol, n-tetradecanol, n-hexadecanol, or n-octadecanol, or a combination comprising one or more of the foregoing.

The cracking can optionally occur in the presence of an added catalyst. Some catalysts include: an aromatic sulfonic acid, sulfuric acid, phosphoric acid, a base (such as sodium hydroxide), a sulfur containing amine compound, a zeolite, sodium hypophosphite, aluminum isopropylate, a sodium salt of one or both of an organic and a mineral acid, or a combination comprising at least one of the foregoing. The catalyst can comprise an aromatic sulfonic acid (such as dodecylbenzene sulfonic acid (DBSA)), sulfuric acid, phosphoric acid, a base (such as sodium hydroxide), or a combination comprising at least one of the foregoing. Optionally, the catalyst can comprise sodium hydroxide. Optionally, the catalyst can comprise an aromatic sulfonic acid; preferably DBSA.

The aromatic sulfonic acid can be represented by the general formula RC₆ H₄ SO₃ H, in which R may be in any position in the phenyl ring. Specifically, the aromatic sulfonic acid can have the Formula (IV) 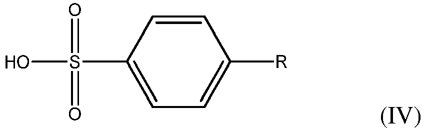 wherein R is a C₁₋₂₅ hydrocarbon group. The C₁₋₂₅ hydrocarbon group can be an alkyl group such as methyl, ethyl, propyl, butyl, pentyl, hexyl, heptyl, octyl, nonyl, undecyl, decyl, dodecyl, octadecyl, nonadecyl, eicosyl, heneicosyl, docosyl, tricosyl, tetracosyl, pentacosyl; an aryl group such as phenyl, tolyl, xylyl, naphthyl, biphenyl, tetraphenyl; an aralkyl group such as benzyl, phenethyl, phenpropyl, phenbutyl, phenhexyl, naphthoctyl; or a cycloalkyl group such as cyclopropyl, cyclobutyl, cyclopentyl, cyclohexyl, cycloheptyl, cyclooctyl. The aromatic sulfonic acid of the Formula I can comprise p-toluenesulfonic acid, dodecylbenzene sulfonic acid, or a combination comprising at least one of the foregoing. The aromatic sulfonic acid can be present in an amount of 100 to 10,000 ppm by weight or 1,000 to 5,000 ppm based on the total weight of the dihydroxy compound.

The sulfur containing amine compound can comprise 2-(4-pyridyl)ethanethiol, 2-mercaptoethylamine, 3-mercaptopropylamine, N,N-dimethyl-3-mercaptopropylamine, N,N-di-n-butyl-4-mercaptobutylamine, and 2,2-dimethylthiazolidine can be used. The sulfur containing amine compound can be used in addition to the aromatic sulfonic acid. The sulfur containing amine compound can be present in an amount of 2 to 30 mole percent (mol%), specifically, 5 to 20 mol% based on the acid group (sulfonic group) in the aromatic sulfonic acid.

The catalyst can comprise a zeolite, for example, in a fixed bed reactor or in a reactive tray in a distillation column. As is used herein, the zeolite comprises an aluminum oxide (i.e., Al₂O₃) and a silicon oxide (SiO₂). A silica to alumina mole ratio can be 2 to 20. The zeolite can be functionalized with a sulfonic group. The zeolite can comprise a metal, for example, cobalt, gallium, copper, zinc, iron, magnesium, manganese, cesium, cerium, or a combination comprising at least one of the foregoing. The metal can be present in one or both of the framework of the zeolite or deposited thereon. The zeolite can be ammonium exchanged. The zeolite can have a pore size of 6 to 15 Angstroms.

The zeolite can comprise a Y zeolite, such as a 13Y zeolite having the formula Na₅₆[(AlO₂)₅₆(SiO₂)₁₃₆]250H₂O. The Y zeolite can have an SiO₂:Al₂O₃ mole ratio of 3 to 6. The Y zeolite can comprise 10 to 14 wt% of Na₂O based on the total weight of the zeolite. The Y zeolite can comprise an ion-exchanged zeolite, for example, with ammonium to result in a Y zeolite comprising less than 1 wt% of Na₂O based on the total weight of the Y zeolite.

The zeolite can comprise a ZSM-5 zeolite. The ZSM-5 zeolite can have the formula 0.9±0.2M_{2/n}O:W₂O₃:5-100YO₂:zH₂O, wherein M is at least one cation; n is the valence thereof; W can comprise aluminum, gallium, or a combination comprising at least one of the foregoing; Y can comprise silicon, germanium, or a combination comprising at least one of the foregoing; and z is 0 to 40. The cation can comprise hydrogen, a rare earth metal, aluminum, a Group II metal, a Group VIII metal, manganese, or a combination comprising at least one of the foregoing. The silica to alumina mole ratio can be 10 to 60.

The zeolite can be present in an amount of 1 to 25 wt%, specifically, 5 to 20 wt% based on the total weight of the dihydroxy compound.

The present method can separate an aromatic alcohol from an overhead stream(s) of a melt polymerization facility. For example, top overhead stream 262, purified aromatic alcohol stream 112, and separation unit stream 132 comprise an aromatic alcohol. The aromatic alcohol can comprise an aromatic alcohol of Formula (III) 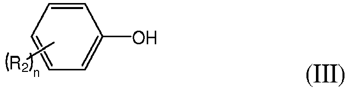 wherein n and R₂ are defined as above in formula (I).

The aromatic alcohol can comprise phenol, o-, m- or p-cresol, dimethylphenol (wherein the methyl groups can be in any desired position on the phenol ring, for example, 2,4-, 2,6- or 3,4-dimethylphenol), o-, m- or p-chlorophenol, o-, m- or p-ethylphenol, o-, m- or p-n-propylphenol), 4-isopropylphenol, 4-n-butylphenol, 4-isobutylphenol, 4-tert-butylphenol, 4-n-pentylphenol, 4-n-hexylphenol, 4-isooctylphenol, 4-n-nonylphenol, o-, m- or p-methoxyphenol, 4-cyclohexylphenol, 4-(1-methyl-1-phenylethyl)-phenol, biphenyl-4-ol, 1-naphthol, 2-naphthol, 4-(1-naphthyl)phenol, 4-(2-naphthyl)phenol, 4-phenoxyphenol, 3-pentadecylphenol, 4-tritylphenol, salicylic acid methyl ester, salicylic acid ethyl ester, salicylic acid n-propyl ester, salicylic acid isopropyl ester, salicylic acid n-butyl ester, salicylic acid isobutyl ester, salicylic acid tert-butyl ester, salicylic acid phenyl ester, salicylic acid benzyl ester, or a combination comprising one or more of the foregoing.

The aromatic alcohol can comprise phenol, 4-tert-butylphenol, biphenyl-4-ol, 4-(1-methyl-1-phenylethyl)-phenol, or a combination comprising one or more of the foregoing.

The aromatic alcohol can be used in a monomer production facility that synthesizes one or both of a diaryl carbonate and a bisphenol.

As used herein, the carbonate compound, also referred to herein as a diaryl carbonate, can have the formula (I) 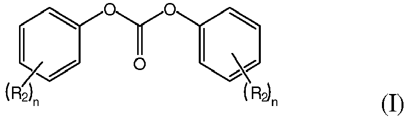 wherein n is an integer 0 to 3 and each R₂ is independently linear or branched; optionally substituted; C₁₋₃₄ alkyl, specifically, C₁₋₆ alkyl, more specifically, C₁₋₄ alkyl; C₁₋₃₄ alkoxy, specifically, C₁₋₆ alkoxy, more specifically, C₁₋₄ alkoxy; C₅₋₃₄ cycloalkyl; C₇₋₃₄ alkylaryl; C₆₋₃₄ aryl; or a halogen radical, specifically, a chlorine radical. R₂ can also represent -COO-R', wherein R' can be H; C₁₋₃₄ alkyl, specifically, C₁₋₆ alkyl, more specifically, C₁₋₄ alkyl; C₁₋₃₄ alkoxy, specifically, C₁₋₁₆ alkoxy, specifically, C₁₋₄ alkoxy; C₅₋₃₄ cycloalkyl; C₇₋₃₄ alkylaryl; or C₆₋₃₄ aryl. The diaryl carbonate can comprise diphenyl carbonate.

The diaryl carbonate of the general formula (I) can comprise diphenyl carbonate, methylphenyl-phenyl carbonates and di-(methylphenyl) carbonates (wherein the methyl group can be in any desired position on the phenyl rings), dimethylphenyl-phenyl carbonates and di-(dimethylphenyl) carbonates (wherein the methyl groups can be in any desired position on the phenyl rings, for example, 2,4-, 2,6-, 3,5- or 3,4-dimethylphenyl), chlorophenyl-phenyl carbonates and di-(chlorophenyl) carbonates (wherein the chloro atom can be in any desired position on the phenyl rings, for example, 2-, 3-, or 4-chlorophenyl), 4-ethylphenyl-phenyl carbonate, di-(4-ethylphenyl) carbonate, 4-n-propylphenyl-phenyl carbonate, di-(4-n-propylphenyl) carbonate, 4-isopropylphenyl-phenyl carbonate, di-(4-isopropylphenyl) carbonate, 4-n-butylphenyl-phenyl carbonate, di-(4-n-butylphenyl) carbonate, 4-isobutylphenyl-phenyl carbonate, di-(4-isobutylphenyl) carbonate, 4-tert-butylphenyl-phenyl carbonate, di-(4-tert-butylphenyl) carbonate, 4-n-pentylphenyl-phenyl carbonate, di-(4-npentylphenyl) carbonate, 4-n-hexylphenyl-phenyl carbonate, di-(4-n-hexylphenyl) carbonate, 4-isooctylphenyl-phenyl carbonate, di-(4-isooctylphenyl) carbonate, 4-n-nonylphenyl-phenyl carbonate, di-(4-n-nonyl-phenyl) carbonate, 4-cyclohexylphenyl-phenyl carbonate, di-(4-cyclohexylphenyl) carbonate, 4-(1-methyl-1-phenylethyl)-phenyl-phenyl carbonate, di-[4-(1-methyl-1-phenylethyl)-phenyl]carbonate, biphenyl-4-yl-phenyl carbonate, di-(biphenyl-4-yl)carbonate, (1-naphthyl)-phenyl carbonate, (2-naphthyl)-phenyl carbonate, di-(1-naphthyl)carbonate, di-(2-naphthyl)carbonate, 4-(1-naphthyl)-phenyl-phenyl carbonate, 4-(2-naphthyl)-phenyl-phenyl carbonate, di-[4-(1-naphthyl)-phenyl] carbonate, di-[4-(2-naphthyl)phenyl] carbonate, 4-phenoxyphenyl-phenyl carbonate, di-(4-phenoxyphenyl) carbonate, 3-pentadecylphenyl-phenyl carbonate, di-(3-pentadecylphenyl) carbonate, 4-tritylphenyl-phenyl carbonate, di-(4-tritylphenyl) carbonate, methyl salicylate-phenyl carbonate, di-(methyl salicylate) carbonate, ethyl salicylate-phenyl carbonate, di-(ethyl salicylate) carbonate, n-propyl salicylate-phenyl carbonate, di-(n-propyl salicylate) carbonate, isopropyl salicylate-phenyl carbonate, di-(isopropyl salicylate) carbonate, n-butyl salicylate-phenyl carbonate, di-(n-butyl salicylate) carbonate, isobutyl salicylate-phenyl carbonate, di-(isobutyl salicylate) carbonate, tert-butyl salicylate-phenyl carbonate, di-(tert-butyl salicylate) carbonate, di-(phenyl salicylate)-carbonate, di-(benzyl salicylate) carbonate, and combinations comprising one or more of the foregoing.

The carbonate compound can comprise less than or equal to 33 parts per billion by weight (ppb), specifically, less than or equal to 20 ppb of molybdenum; less than or equal to 33 ppb, specifically, less than or equal to 20 ppb vanadium; less than or equal to 33 ppb, specifically, less than or equal to 20 ppb chromium; less than or equal to 75 ppb, specifically, less than or equal to 50 ppb titanium; less than or equal to 375 ppb, specifically, less than or equal to 250 ppb of niobium; less than or equal to 33 ppb, specifically, less than or equal to 20 ppb of nickel; less than or equal to 10 ppb, specifically, less than or equal to 5 ppb zirconium; less than or equal to 10 ppb, specifically, less than or equal to 5 ppb of iron, or a combination comprising one or more of the foregoing; all based on the total weight of the carbonate compound.

The onsite monomer production facility can comprise a diaryl carbonate production facility. There are several methods by which diaryl carbonate can be produced in the monomer production facility.

The diaryl carbonate can be prepared by reacting the aromatic hydroxy compound and carbon monoxide in the presence of oxygen, where the reaction can be facilitated by a catalyst and an optional organic salt. For example, the reaction can be the oxidative carbonylation of phenol, where the reaction can occur in a fixed-bed reactor or in an autoclave reactor. Suitable catalysts for the oxidative carbonylation of aromatic hydroxy compounds include a palladium catalyst. The palladium catalyst can be in solvated form (such as PdBr₂ promoted with transition metal oxides and solvated promoters, including one or more of N(Bu)₄Br, Mn(AcAc)₂, NaO(C₆H₅), and the like), suspended form with Pd supported on pulverized TiO₂, or extrudate form with Pd supported on rare earth metal oxide. The palladium catalyst can comprise Pd(OAc)₂/hydrotalcite. As used herein, Bu means butyl, AcAc means acetylacetonate, and OAc means acetate. The catalyst can comprise a cocatalyst, such as a cesium compound, a manganese compound, a cobalt compound, a copper compound, hydroquinone, benzoquinone, naphthoquinone, or a combination comprising one or more of the foregoing. The organic salt can comprise, for example, ⁿBu₄NBr, ⁿBu₄PBr, PPNBr, and the like.

The diaryl carbonate can be prepared by reacting the aromatic alcohol with phosgene, for example, in either the gas or liquid phase; or the diaryl carbonate can be prepared by reacting the aromatic alcohol with a dialkyl carbonate; where said reactions can occur in the presence of a transesterification catalyst. The aromatic alcohol and phosgene and/or the dialkyl carbonate can be added in a molar ratio of 1:0.1 to 1:10, specifically, 1:0.2 to 1:5, more specifically, 1:0.5 to 1:3 (aromatic alcohol to phosgene and/or the dialkyl carbonate). The indicated molar ratio does not take into account any recycled components that can be added back to the production column.

The dialkyl carbonate can comprise the dialkyl carbonate of formula (II) 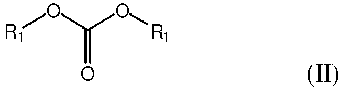 wherein each R₁ independently is linear or branched; optionally substituted; C₁₋₃₄ alkyl, specifically, C₁₋₆ alkyl, more specifically, C₁₋₄ alkyl. The C₁₋₄ alkyl can comprise methyl, ethyl, n-propyl, isopropyl, n-butyl, sec-butyl, tert-butyl, or a combination comprising one or more of the foregoing. The C₁₋₆ alkyl can comprise n-pentyl, 1-methylbutyl, 2-methylbutyl, 3-methylbutyl, neopentyl, 1-ethylpropyl, cyclohexyl, cyclopentyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, or a combination comprising one or more of the foregoing. The C₁-C₃₄-alkyl can comprise n-heptyl, n-octyl, pinacyl, adamantyl, an isomeric menthyl, n-nonyl, n-decyl, n-dodecyl, n-tridecyl, n-tetradecyl, n-hexadecyl, or n-octadecyl, or a combination comprising one or more of the foregoing. The dialkyl carbonate can comprise dimethyl carbonate, diethyl carbonate, dipropyl carbonate (e.g., di(n-propyl) carbonate, and/or di(isopropyl) carbonate), dibutyl carbonate (e.g., di(n-butyl) carbonate, di(sec-butyl) carbonate, and/or di(tert-butyl) carbonate), dihexyl carbonate, or a combination comprising one or more of the foregoing.

A catalyst can be used to facilitate the reaction between the aromatic alcohol and either phosgene or the dialkyl carbonate. The catalyst can be a homogeneous catalyst and/or a heterogeneous catalyst, wherein a heterogeneous catalyst comprises two or more catalysts. The catalyst can comprise hydrides, oxides, hydroxides, alcoholates, amides and other salts of alkali and alkaline earth metals, such as of lithium, sodium, potassium, rubidium, cesium, magnesium and calcium, specifically, lithium, sodium, potassium, magnesium, calcium, or a combination comprising one or more of the foregoing. The catalyst, when homogeneous, can be introduced to the reaction mixture in dissolved or suspended form together with the stream containing the aromatic alcohol. Alternatively, the catalyst can be introduced, for example, in the reaction alcohol or a suitable inert solvent. A heterogeneous catalyst can be used in a packed bed, a column, or in special catalytic distillation arrangements, as well as in other arrangements.

The onsite monomer production facility can comprise a bisphenol production facility. There are several methods by which the bisphenol can be produced in the monomer production facility. The bisphenol can be formed by reacting the aromatic alcohol, and one or both of a ketone and an aldehyde in the presence of an ion exchange resin. A mole ratio of the aromatic alcohol, to the aldehyde and/or ketone can be 4 to 65, specifically, 7 to 15. The ketone can comprise 9-fluorenone, benzophenone, acetone, acetophenone, cyclohexanone, 3,3,5-trimethylcyclohexanone, 4-hydroxyacetophenone, 4,4'-dihydroxybenzophenone, or a combination comprising at least one of the foregoing. The aldehyde can comprise formaldehyde, acetaldehyde, propionaldehyde, butyraldehyde, or a combination comprising at least one of the foregoing. Water can be present in the reaction in an amount of 0 to 5 wt% based on the total weight of the reaction mixture. The bisphenol formation reaction can occur at a temperature of 10 to 100°C, specifically, 40 to 90°C.

The bisphenol can be formed by reacting the aromatic alcohol and ketone in the presence of an ion exchange resin, where the reaction mixture can comprise less than or equal to 100 ppm, specifically, less than or equal to 10 ppm, more specifically, less than or equal to 1 ppm of the aldehyde based on the total weight of the ketone.

The ketone and/or aldehyde can comprise less than or equal to 250 ppm, specifically, less than or equal to 225ppm, and more specifically, less than or equal to 200 ppm of an C₁₋₄ alkyl alcohol (such as methanol and/or ethanol) based on the total weight of the ketone and/or aldehyde. The ketone and/or aldehyde can comprise less than or equal to 100 ppm, specifically, 0 to 10 ppm, and more specifically, 0 to 1 ppm of methanol based on the total weight of the ketone and/or aldehyde.

The alcohol level in the ketone can be reduced, for example, by reacting the alcohol (for example, methanol) with an amount of a diaryl carbonate such as a diaryl carbonate of formula (I), for example, diphenyl carbonate, bismethyl salicyl carbonate, an activated diaryl carbonate, and the like, to yield an aryl alkyl carbonate and a hydroxy compound. For example, methanol present in the ketone can react with diphenyl carbonate to form phenyl methyl carbonate and phenol. This reaction can be performed in the presence of a transesterification catalyst. As the reaction products, for example, phenyl methyl carbonate and phenol, are less volatile than methanol, they are more easily separated from the ketone, for example, by distillation or flash separation. The alcohol reduction reaction can be performed at a molar ratio of diaryl carbonate to alcohol of greater than or equal to 1, specifically, greater than or equal to 2, more specifically, greater than or equal to 5, more specifically, greater than or equal to 10. The alcohol reduction reaction can be performed at a temperature of greater than or equal to 50°C, specifically, greater than or equal to 100°C, more specifically, greater than or equal to 130°C, even more specifically, greater than or equal to 145°C.

The ion exchange resin can comprise a cross-linked polystyrene resin that is functionalized to have acid sites. The acid sites can comprise a sulfonic acid functionality, a phosphonic acid functionality, a carboxylic acid functionality, or a combination comprising at least one of the foregoing. The ion exchange resin can have an acidic milliequivalent per gram catalyst (meq/g) value (proton exchange capacity), prior to modification, of greater than or equal to 3.5 meq/g, specifically, 4 to 10 meq/g, more specifically, 5 to 10 meq/g when dry. The polystyrene resin can be cross-linked, for example, with a polycyclic aromatic divinyl monomer, a divinyl benzene, a divinyl toluene, a divinyl biphenyl monomer, or a combination comprising at least one of the foregoing.

Examples of ion exchange resins include DIAION™ SK104, DIAION™ SK1B, DIAION™ PK208, DIAION™ PK212 and DIAION™ PK216 (manufactured by Mitsubishi Chemical Industries, Limited), A-121, A-232, and A-131, (manufactured by Rohm & Haas), T-38, T-66 and T-3825 (manufactured by Thermax), Lewatit K1131, Lewatit K1221 (manufactured by Bayer), DOWEX™ 50W2X, DOWEX™ 50W4X, DOWEX™ 50W8X resins (manufactured by Dow Chemical), Indion 180, Indion 225 (manufactured by Ion Exchange India Limited), and Purolite CT-222 and Purolite CT-122 (manufactured by Purolite).

The ion exchange resin catalyst can be used in combination with a bulk promoter or an attached promoter. The bulk promoter can comprise a cycloaliphatic thiol such as cyclohexanethiol and cyclopentanethiol; an alkyl thiol such as methyl mercaptan and ethyl mercaptan; aromatic thiols such as thiophenol and benzylthiol; and aliphatic thiols such as butanethiol, hexanethiol, octadecanethiol, thioglycollic acid, and 3-mercaptopropionic acid; or a combination comprising at least one of the foregoing. The attached promoter can comprise a silylmethanethiol, 2-mercaptomethylpyridine, 4-pyridylethylmercaptan, cysteamine, 4-aminobutanethiol immobilized in an amine modified acidic resin catalyst, or a combination comprising at least one of the foregoing.

Set forth below are non-limiting examples.

### EXAMPLES

### Example 1: Hydrolysis with sodium hydroxide (NaOH) as the catalyst.

Hydrolysis was performed using 750 parts per million by weight (ppm) sodium hydroxide (NaOH) in unit 136. Mixing and cracking unit 136 was operated at 180°C and 25 bars (2,500 kPa) in liquid phase. The compositions of the streams are set forth in Table 1. As can be seen in Table 1, the use of NaOH in the unit 136 was effective at converting DPC to phenol (PhOH) and carbon dioxide (CO₂), and converting carbonates (PC oligomers) to BPA and CO₂. However, if cracking of the BPA is desired, an additional cracking unit and/or another catalyst are needed.

### Example 2: Hydrolysis with DBSA as the catalyst.

Hydrolysis was performed using 6,000 ppm DBSA in unit 136. Mixing and cracking unit 136 was operated at 180°C and 25 bars (2,500 kPa) in liquid phase. The compositions of the streams are set forth in Table 2. As can be seen in Table 2, the use of DBSA in the unit 136 was effective at converting DPC to phenol and CO₂, converting carbonates (PC oligomers) to BPA and CO₂, and in converting BPA to additional phenol and byproducts (e.g., p-isopropyl phenol (IPP)).

### Example 3: Methanolysis with no catalyst.

Methanolysis was performed with no catalyst being added to unit 136. Mixing and cracking unit 136 was operated at 180°C and 25 bars (2,500 kPa) in liquid phase. The compositions of the streams are set forth in Table 3. As can be seen in Table 3, with no catalyst added to unit 136, alkyl alcohol (methanol) was effective at converting DPC to phenol (PhOH) and carbon dioxide (CO₂), and converting carbonates (PC oligomers) to BPA and CO₂. However, if cracking of the BPA is desired, an additional cracking unit and/or a catalyst are needed.

### Example 4: Methanolysis with DBSA as the catalyst.

Methanolysis was performed using 6,000 ppm DBSA in unit 136. Mixing and cracking unit 136 was operated at 180°C and 25 bars (2,500 kPa) in liquid phase. The compositions of the streams are set forth in Table 4. As can be seen in Table 4, the use of DBSA in the unit 136 was effective at converting DPC to phenol and CO₂, converting carbonates (PC oligomers) to BPA and CO₂, and in converting BPA to additional phenol and byproducts (e.g., p-isopropyl phenol (IPP)).

Comparing Examples 3 and 4, it can be seen that a lower amount of BPA remained in stream 138 with the use of DBSA. Comparing the use of DBSA via hydrolysis versus methanolysis, it can be seen that with the methanolysis, a greater amount of the DPC was cracked.

From the examples, it is understood that, depending upon the desired end product, the cracking agent, the use of a catalyst, and the type of catalyst can be chosen. Unexpectedly, alkyl alcohol cracking agent is effective at recovering phenol without the use of a catalyst. However, a dialkyl carbonate is also produced. Hydrolysis with the use of a catalyst, e.g., a base, can be used to recover phenol without the production of a dialkyl carbonate.

The use of alkyl alcohol cracking agent in combination with acid cracking can be used to further crack BPA and even the DMC for further phenol recovery. For example, the use of alkyl alcohol cracking agent in combination with an aromatic sulfonic acid (e.g., dodecylbenzene sulfonic acid) can be used to further crack BPA and even the DMC for further phenol recovery.

For any of these reactions, the products and/or reactants of the reactions (aromatic alcohol (e.g., PhOH), dialkyl carbonate (e.g., DMC), BPA, alkyl alcohol (e.g., MeOH), can be separated into two or more streams in unit 130. The use of side draw outlets (e.g. dashed lines in FIG. 2) enables further separation of the streams without a further column.

**Table 1**

| STREAM | 114 | 126+122 | 126 | 122 | 124 | 128 | 138 | *132* | *134* |
|---|---|---|---|---|---|---|---|---|---|
| Mass flow, kg/h | 650.0 | 377.0 | 56.6 | 320.5 | 273.0 | 118.3 | 391.3 | *239.5* | *151.8* |
| PhOH, wt% | 1.5 | 0.9 | 0.5 | 0.6 | 0.0 | | 35.0 | *56.6* | *0.9* |
| BPA, wt% | 0.0 | 0.0 | 0.0 | 0.0 | 0.0 | | 30.3 | *0.0* | *78.2* |
| DPC, wt% | 58.2 | 95.3 | 73.1 | 97.5 | 29.7 | | 1.3 | *2.1* | *0.2* |
| DMC, % | | | | | | | | *0.0* | *0.0* |
| H₂O, wt% | | | | | | 100.0 | 24.2 | *39.5* | *0.0* |
| MeOH, wt% | | | | | | | | *0.0* | *0.0* |
| Others, wt% | 40.3 | 3.9 | 26.4 | 1.9 | 70.3 | | 9.13 | *1.79* | *20.7* |
| PhOH, kg/h | 10.0 | 3.3 | 0.3 | 1.8 | 0.1 | 0.0 | 137.0 | *135.7* | *1.4* |
| BPA, kg/h | 0.0 | 0.0 | 0.0 | 0.0 | 0.0 | 0.0 | 118.7 | | *118.7* |
| DPC, kg/h | 378.3 | 359.1 | 41.4 | 312.5 | 81.0 | 0.0 | 5.2 | *4.9* | *0.3* |
| DMC, kg/h | | | | | | 0.0 | 0.0 | | *0.0* |
| H₂O, kg/h | | | | | | 118.3 | 94.6 | *94.6* | *0.0* |
| MeOH, kg/h | | | | | | 0.0 | 0.0 | | *0.0* |
| Others, kg/h | 261.6 | 14.6 | 14.9 | 6.1 | 191.9 | 0.0 | 35.7 | *4.3* | *31.5* |

**Table 2**

| STREAM | 114 | 126+122 | 126 | 122 | 124 | 128 | 138 | *132* | *134* |
|---|---|---|---|---|---|---|---|---|---|
| Mass flow, kg/h | 650.0 | 377.0 | 56.6 | 320.5 | 273.0 | 118.3 | 391.3 | *250.9* | *140.4* |
| PhOH, wt% | 1.5 | 0.9 | 0.5 | 0.6 | 0.0 | | 23.6 | *36.5* | *0.7* |
| BPA, wt% | 0.0 | 0.0 | 0.0 | 0.0 | 0.0 | | 11.8 | *0.0* | *32.9* |
| DPC, wt% | 58.2 | 95.3 | 73.1 | 97.5 | 29.7 | | 11.5 | *17.0* | *1.6* |
| DMC, % | | | | | | | | *0.0* | *0.0* |
| H₂O, wt% | | | | | | 100.0 | 27.2 | *42.4* | *0.0* |
| MeOH, wt% | | | | | | | | *0.0* | *0.0* |
| Others, wt% | 40.3 | 3.9 | 26.4 | 1.9 | 70.3 | | 25.86 | *4.03* | *64.88* |
| PhOH, kg/h | 10.0 | 3.3 | 0.3 | 1.8 | 0.1 | 0.0 | 92.5 | *91.6* | *0.9* |
| BPA, kg/h | 0.0 | 0.0 | 0.0 | 0.0 | 0.0 | 0.0 | 46.1 | | *46.1* |
| DPC, kg/h | 378.3 | 359.1 | 41.4 | 312.5 | 81.0 | 0.0 | 45.0 | *42.7* | *2.2* |
| DMC, kg/h | | | | | | | | | *0.0* |
| H₂O, kg/h | | | | | | 118.3 | 106.5 | *106.5* | *0.0* |
| MeOH, kg/h | | | | | | | | | *0.0* |
| Others, kg/h | 261.6 | 14.6 | 14.9 | 6.1 | 191.9 | | 101.2 | *10.1* | *91.1* |

**Table 3**

| STREAM | 114 | 126+122 | 126 | 122 | 124 | 128 | 138 | *132* | *134* |
|---|---|---|---|---|---|---|---|---|---|
| Mass flow, kg/h | 650.0 | 377.0 | 56.6 | 320.5 | 273.0 | 291.2 | 564.2 | *430.5* | *133.7* |
| PhOH, wt% | 1.5 | 0.9 | 0.5 | 0.6 | 0.0 | | 22.0 | *28.5* | *0.9* |
| BPA, wt% | 0.0 | 0.0 | 0.0 | 0.0 | 0.0 | | 17.8 | *0.0* | *75.1* |
| DPC, wt% | 58.2 | 95.3 | 73.1 | 97.5 | 29.7 | | 0.4 | *0.5* | *0.1* |
| DMC, % | | | | | | | 35.6 | *39.9* | *0.6* |
| H₂O, wt% | | | | | | | | *0.0* | *0.0* |
| MeOH, wt% | | | | | | | 23.5 | *30.6* | *0.5* |
| Others, wt% | 40.3 | 3.9 | 26.4 | 1.9 | 70.3 | | 5.66 | *0.37* | *22.71* |
| PhOH, kg/h | 10.0 | 3.3 | 0.3 | 1.8 | 0.1 | | 124.1 | *122.9* | *1.2* |
| BPA, kg/h | 0.0 | 0.0 | 0.0 | 0.0 | 0.0 | | 100.4 | | *100.4* |
| DPC, kg/h | 378.3 | 359.1 | 41.4 | 312.5 | 81.0 | | 2.3 | *2.1* | *0*.*1* |
| DMC, kg/h | | | | | | 87.4 | 172.9 | *172.0* | *0.9* |
| H₂O, kg/h | | | | | | | | | *0.0* |
| MeOH, kg/h | | | | | | 203.8 | 132.6 | *131.9* | *0.7* |
| Others, kg/h | 261.6 | 14.6 | 14.9 | 6.1 | 191.9 | | 31.9 | *1.6* | *30.4* |

**Table 4**

| STREAM | 114 | 126+122 | 126 | 122 | 124 | 128 | 138 | *132* | *134* |
|---|---|---|---|---|---|---|---|---|---|
| Mass flow, kg/h | 650.0 | 377.0 | 56.6 | 320.5 | 273.0 | 291.2 | 564.2 | *394.0* | *170.2* |
| PhOH, wt% | 1.5 | 0.9 | 0.5 | 0.6 | 0.0 | | 18.0 | *25.5* | *0.6* |
| BPA, wt% | 0.0 | 0.0 | 0.0 | 0.0 | 0.0 | | 10.4 | *0.0* | *34.5* |
| DPC, wt% | 58.2 | 95.3 | 73.1 | 97.5 | 29.7 | | 1.7 | *2.3* | *0.3* |
| DMC, % | | | | | | | 28.6 | *33.7* | *0.4* |
| H₂O, wt% | | | | | | | | *0.0* | *0.0* |
| MeOH, wt% | | | | | | | 26.0 | *37.0* | *0.4* |
| Others, wt% | 40.3 | 3.9 | 26.4 | 1.9 | 70.3 | | 20.26 | *1.45* | *63.82* |
| PhOH, kg/h | 10.0 | 3.3 | 0.3 | 1.8 | 0.1 | | 101.6 | *100.5* | *1.0* |
| BPA, kg/h | 0.0 | 0.0 | 0.0 | 0.0 | 0.0 | | 58.7 | | *58.7* |
| DPC, kg/h | 378.3 | 359.1 | 41.4 | 312.5 | 81.0 | | 9.6 | *9.1* | *0.5* |
| DMC, kg/h | | | | | | 87.4 | 133.4 | *132.7* | *0.7* |
| H₂O, kg/h | | | | | | | | | *0.0* |
| MeOH, kg/h | | | | | | 203.8 | 146.7 | *146.0* | *0.7* |
| Others, kg/h | 261.6 | 14.6 | 14.9 | 6.1 | 191.9 | | 114.3 | *5.7* | *108.6* |

The above method and system for recovering an aromatic alcohol is further described in the below embodiments.
Embodiment 1: A method of purifying an aromatic alcohol comprising: melt polymerizing a dihydroxy compound and a carbonate compound in the presence of a transesterification catalyst to form the aromatic alcohol and a polycarbonate in a melt polymerization facility; removing the aromatic alcohol in an overhead stream; separating the overhead stream in an aromatic alcohol purification unit into a purified aromatic alcohol stream comprising the aromatic alcohol and a purification unit bottom stream; separating the purification unit bottom stream in a diarylcarbonate purification unit into a diaryl carbonate stream and a diaryl carbonate purification unit bottom stream; and cracking the diaryl carbonate purification unit bottom stream in a cracker unit in the presence of a cracking agent to form a stream comprising additional aromatic alcohol.
Embodiment 2: The method of Embodiment 1, further comprising one or both of directing the purified aromatic alcohol stream to a monomer production facility that is in fluid communication with the aromatic alcohol purification unit and directing the separation unit stream to the monomer production facility that is in fluid communication with the cracker unit.
Embodiment 3: The method of Embodiment 2, further comprising forming a diaryl carbonate in the monomer production facility by reacting one or both of the aromatic alcohol and the additional aromatic alcohol with carbon monoxide in the presence of oxygen and a carbonylation catalyst to form the diaryl carbonate; or reacting one or both of the aromatic alcohol and the additional aromatic alcohol with phosgene and/or a dialkyl carbonate in the presence of a second transesterification catalyst to form the diaryl carbonate.
Embodiment 4: The method of Embodiment 3, further comprising adding the diaryl carbonate to the melt polymerization facility.
Embodiment 5: The method of any one of Embodiments 2-4, further comprising forming a bisphenol in the monomer production facility by reacting one or both of the aromatic alcohol and the additional aromatic alcohol with a ketone and/or an aldehyde in the presence of an ion exchange resin to form the bisphenol; preferably, wherein the additional aromatic alcohol has a content of less than or equal to 10 ppm of C₁₋₄ alkyl alcohol, and less than or equal to 10 ppm of C₁₋₄ dialkyl carbonate.
Embodiment 6: The method of Embodiment 5, wherein the reacting is with the ketone; and wherein the aldehyde is present during the reacting in an amount of less than or equal to 100 ppm, specifically, less than or equal to 10 ppm, more specifically, less than or equal to 1 ppm based on the total weight of the ketone.
Embodiment 7: The method of any one of Embodiments 5-6, wherein a C₁₋₄ alkyl alcohol is present during the reacting in an amount of less than or equal to 100 ppm, specifically, 0 to 10 ppm, and more specifically, 0 to 1 ppm based on the total weight of the ketone and aldehyde.
Embodiment 8: The method of any one of Embodiments 5-7, further comprising adding the bisphenol to the melt polymerization facility.
Embodiment 9: The method of any one of the preceding embodiments, comprising removing the purified aromatic alcohol stream as a side stream from the aromatic alcohol purification unit and removing a light boiler top stream from a top of the aromatic alcohol purification unit.
Embodiment 10: The method of any one of the preceding embodiments, comprising removing the diaryl carbonate stream as a side stream from the diaryl carbonate purification unit and removing a medium boiler top stream from a top of the diaryl carbonate purification unit.
Embodiment 11: The method of any one of the preceding embodiments, wherein the purified aromatic alcohol stream 112 comprises greater than or equal to 99 wt%, specifically, 99.5 to 100 wt%, more specifically, 99.7 to 100 wt% aromatic alcohol based on the total weight of the purified aromatic alcohol stream.
Embodiment 12: The method of any one of the preceding embodiments, wherein the cracking occurs at one or more of a temperature of 100 to 500°C, specifically, 140 to 250°C; a pressure of 100 to 1,000 kPa, specifically, 100 to 250 kPa; and a residence time in the cracker of 0.2 to 20 hours, or 0.5 to 10 hours.
Embodiment 13: The method of any one of the preceding embodiments, wherein the cracking is further in the presence of a catalyst, and wherein the catalyst comprises at least one of an aromatic sulfonic acid, a sulfur containing amine compound, a zeolite, sodium hypophosphite, aluminum isopropylate, a sodium salt of one or both of an organic and a mineral acid, and a base; preferably wherein the catalyst comprises at least one of sodium hydroxide and dodecylbenzene sulfonic acid, more preferably wherein the catalyst comprises dodecylbenzene sulfonic acid.
Embodiment 14: The method of any one of embodiments 1 - 13, wherein none of an aromatic sulfonic acid, sulfuric acid, phosphoric acid, sodium hydroxide, a sulfur containing amine compound, a zeolite, sodium hypophosphite, aluminum isopropylate, a sodium salt of one or both of an organic and a mineral acid, is added to the cracking unit; preferably wherein no catalyst is added to the cracking unit.
Embodiment 15: The method of any one of the preceding embodiments, wherein one or both of the carbonate compound and the diaryl carbonate each independently comprise less than or equal to 33 ppb of molybdenum; less than or equal to 33 ppb of vanadium; less than or equal to 33 ppb of chromium; less than or equal to 75 ppb of titanium; less than or equal to 375 ppb of niobium; less than or equal to 33 ppb of nickel; less than or equal to 10 ppb of zirconium; less than or equal to 10 ppb of iron, or a combination comprising one or more of the foregoing; all based on the total weight of the respective carbonate compound or diaryl carbonate.
Embodiment 16: The method of any one of the preceding embodiments, wherein the melt polymerizing comprises consecutively polymerizing in a first oligomerization unit; polymerizing in a second oligomerization unit that is in fluid communication with the first oligomerization unit; polymerizing in a first polymerization unit that is in fluid communication with the second oligomerization unit; and polymerizing in a second polymerization unit that is in fluid communication with the first polymerization unit to form the polycarbonate; wherein the one or more overhead streams is withdrawn from one or more of the second oligomerization unit, the first polymerization unit, and the second polymerization unit.
Embodiment 17: The method of Embodiment 16, further comprising directing a first oligomerization overhead stream from the first oligomerization unit to an oligomerization overhead purification column; and directing a top overhead stream from the oligomerization overhead purification column to a monomer production facility that is in fluid communication with the oligomerization overhead purification column, for example, the monomer production facility of any one of Embodiments 2-8.
Embodiment 18: The method of any one of the preceding embodiments, wherein the diaryl carbonate purification unit bottom stream and a cracking agent are added to the cracking unit; preferably wherein the cracking agent comprises at least one of water and an alkyl alcohol; preferably wherein the alkyl alcohol is methanol.
Embodiment 19: The method of any one of the preceding embodiments, further comprising adding the cracking agent to a mixing unit located upstream of the cracker unit.
Embodiment 20: The method of any one of the preceding embodiments, wherein the diaryl carbonate purification unit bottom stream and a cracking agent are added to the cracking unit; preferably wherein the cracking agent comprises at least one of water and an alkyl alcohol; preferably wherein the alkyl alcohol is methanol.
Embodiment 21: The method of any one of the preceding embodiments, wherein the resultant stream further comprises alkyl carbonate; preferably wherein the alkyl carbonate comprises dimethyl carbonate.
Embodiment 22: The method of any one of the preceding embodiments, wherein the resultant stream comprises an aromatic alcohol stream and an alkyl carbonate stream, and wherein the aromatic alcohol stream and the alkyl carbonate stream are separately removed; preferably wherein the resultant stream is separated in a separation unit into the aromatic alcohol stream and the alkyl carbonate stream and a bottom stream.
Embodiment 23: A system for purifying an overhead stream from a melt polymerization facility, comprising: an aromatic alcohol purification unit in fluid communication with the overhead stream from the melt polymerization facility, wherein the aromatic alcohol purification unit is capable of separating a purified aromatic alcohol stream and a purification unit bottom stream; a diarylcarbonate purification unit that is in fluid communication with the aromatic alcohol purification unit via the purification unit bottom stream, wherein diarylcarbonate purification unit is capable of separating a diaryl carbonate stream and a diaryl carbonate purification unit bottom stream; and a cracker unit that is in fluid communication with the diarylcarbonate purification unit via the diaryl carbonate purification unit bottom stream and a cracking agent stream, wherein the cracker unit is capable of forming a resultant stream comprising an additional aromatic alcohol. The cracker unit can be capable of forming a separation unit stream comprising an additional aromatic alcohol and a heavy boiler bottom stream; or the cracker unit can be in fluid communication with a separation unit and where the separation unit (which is at least one unit, preferably 2 units, more preferably greater than 2 units) is capable of forming a heavy boiler bottom stream, and separation unit stream(s). At least one of the separation unit streams comprises an additional aromatic alcohol, and preferably at least one of the separation unit streams comprises alkyl carbonates.
Embodiment 24: The system of Embodiment 23, wherein at least one of the aromatic alcohol purification unit, the cracker unit, and the separation unit are in fluid communication with a monomer production facility that is capable of forming one or both of a diaryl carbonate and a bisphenol.
Embodiment 25: The system of any one of Embodiments 23 - 24, wherein the melt polymerization facility comprises a first oligomerization unit; a second oligomerization unit that is in fluid communication with the first oligomerization unit via a first oligomerized stream; a first polymerization unit that is in fluid communication with the second oligomerization unit via a second oligomerized stream; and a second polymerization that is in fluid communication with the first polymerization unit via first polymerized stream; wherein the aromatic alcohol purification unit is in fluid communication with an overhead stream from one or more of the second oligomerization unit, the first polymerization unit, and the second polymerization.
Embodiment 26: The system of Embodiment 25, wherein the first oligomerization unit is in fluid communication with oligomerization overhead purification column via first oligomerization overhead stream; and wherein oligomerization overhead purification column is in fluid communication with a monomer production facility that is capable of forming one or both of a diaryl carbonate and a bisphenol.
Embodiment 27: The system of any of Embodiments 23-26, further comprising a separation unit in fluid communication with the cracking unit via the resultant stream, wherein the separation unit is capable of separating the resultant stream into an aromatic alcohol stream, an alkyl carbonate stream, and a bottom stream; preferably wherein the separation unit comprises greater than or equal to two distillation units; or wherein the cracking unit is capable of separating the resultant stream into an aromatic alcohol stream, an alkyl carbonate stream, and a bottom stream.

In general, this disclosure can alternately comprise, consist of, or consist essentially of, any appropriate components herein disclosed. This disclosure can additionally, or alternatively, be formulated so as to be devoid, or substantially free, of any components, materials, ingredients, adjuvants or species used in the prior art compositions or that are otherwise not necessary to the achievement of the function and/or objectives of the present disclosure.

All ranges disclosed herein are inclusive of the endpoints, and the endpoints are independently combinable with each other (e.g., ranges of "up to 25 wt%, or, more specifically, 5 to 20 wt%", is inclusive of the endpoints and all intermediate values of the ranges of "5 to 25 wt%," etc.). "Combination" is inclusive of blends, mixtures, alloys, reaction products, and the like. Furthermore, the terms "first," "second," and the like, herein do not denote any order, quantity, or importance, but rather are used to denote one element from another. The terms "a" and "an" and "the" herein do not denote a limitation of quantity, and are to be construed to cover both the singular and the plural, unless otherwise indicated herein or clearly contradicted by context. "Or" means "and/or." The suffix "(s)" as used herein is intended to include both the singular and the plural of the term that it modifies, thereby including one or more of that term (e.g., the film(s) includes one or more films). Reference throughout the specification to "one embodiment," "another embodiment," "an embodiment," and so forth, means that a particular element (e.g., feature, structure, and/or characteristic) described in connection with the embodiment is included in at least one embodiment described herein, and may or may not be present in other embodiments. "Optional" or "optionally" means that the subsequently described event or circumstance can or cannot occur, and that the description includes instances where the event occurs and instances where it does not. Unless defined otherwise, technical and scientific terms used herein have the same meaning as is commonly understood by one of skill in the art to which this disclosure belongs. Unless otherwise stated, test standards are the most recent as of the filing date of the application.

## Claims

1. A method of purifying an aromatic alcohol comprising:
melt polymerizing a dihydroxy compound and a carbonate compound in the presence of a transesterification catalyst to form the aromatic alcohol and a polycarbonate in a melt polymerization facility;
removing the aromatic alcohol in an overhead stream;
separating the overhead stream in an aromatic alcohol purification unit into a purified aromatic alcohol stream comprising the aromatic alcohol and a purification unit bottom stream;
separating the purification unit bottom stream in a diary carbonate purification unit into a diaryl carbonate stream and a diaryl carbonate purification unit bottom stream; and
cracking a compound in the diaryl carbonate purification unit bottom stream in a cracker unit in the presence of a cracking agent to form a resultant stream comprising additional aromatic alcohol.

2. The method of Claim 1, further comprising one or both of directing the purified aromatic alcohol stream to a monomer production facility that is in fluid communication with the aromatic alcohol purification unit and directing the separation unit stream to the monomer production facility that is in fluid communication with the cracker unit.

3. The method of Claim 2, further comprising forming a diaryl carbonate in the monomer production facility by
reacting one or both of the aromatic alcohol and the additional aromatic alcohol with carbon monoxide in the presence of oxygen and a carbonylation catalyst to form the diaryl carbonate; or
reacting one or both of the aromatic alcohol and the additional aromatic alcohol with phosgene and/or a dialkyl carbonate in the presence of a second transesterification catalyst to form the diaryl carbonate.

4. The method of any one of Claims 2-3, further comprising forming a bisphenol in the monomer production facility by
reacting one or both of the aromatic alcohol and the additional aromatic alcohol with a ketone and/or an aldehyde in the presence of an ion exchange resin to form the bisphenol;
preferably wherein the additional aromatic alcohol has a content of less than or equal to 10 ppm of C₁₋₄ alkyl alcohol, and less than or equal to 10 ppm of C₁₋₄ dialkyl carbonate.

5. The method of any one of the preceding claims, wherein the resultant stream further comprises alkyl carbonate; preferably wherein the alkyl carbonate comprises dimethyl carbonate; and/or
wherein the resultant stream comprises an aromatic alcohol stream and an alkyl carbonate stream, and wherein the aromatic alcohol stream and the alkyl carbonate stream are separately removed; preferably wherein the resultant stream is separated in a separation unit into the aromatic alcohol stream, the alkyl carbonate stream and a bottom stream.

6. The method of any one of the preceding claims, comprising removing the diaryl carbonate stream as a side stream from the diaryl carbonate purification unit and removing a medium boiler top stream from a top of the diaryl carbonate purification unit.

7. The method of any one of the preceding claims, wherein the purified aromatic alcohol stream comprises greater than or equal to 99 wt%, specifically, 99.5 to 100 wt%, more specifically, 99.7 to 100 wt% aromatic alcohol based on the total weight of the purified aromatic alcohol stream.

8. The method of any one of the preceding claims, wherein the cracking occurs at one or more of a temperature of 100 to 500°C, specifically, 140 to 250°C; a pressure of 100 to 1,000 kPa, specifically, 100 to 250 kPa; and a residence time in the cracker of 0.2 to 20 hours, or 0.5 to 10 hours.

9. The method of any one of the preceding claims, wherein the cracking is further in the presence of a catalyst, and wherein the catalyst comprises at least one of an aromatic sulfonic acid, a sulfur containing amine compound, a zeolite, sodium hypophosphite, aluminum isopropylate, a sodium salt of one or both of an organic and a mineral acid, and a base; preferably wherein the catalyst comprises at least one of sodium hydroxide and dodecylbenzene sulfonic acid, more preferably wherein the catalyst comprises dodecylbenzene sulfonic acid.

10. The method of any one of the preceding claims, wherein the diaryl carbonate purification unit bottom stream and a cracking agent are added to the cracking unit; preferably wherein the cracking agent comprises at least one of water and an alkyl alcohol; preferably wherein the alkyl alcohol is methanol.

11. The method of any one of the preceding claims, wherein one or both of the carbonate compound and the diaryl carbonate each independently comprise less than or equal to 33 ppb of molybdenum; less than or equal to 33 ppb of vanadium; less than or equal to 33 ppb of chromium; less than or equal to 75 ppb of titanium; less than or equal to 375 ppb of niobium; less than or equal to 33 ppb of nickel; less than or equal to 10 ppb of zirconium; less than or equal to 10 ppb of iron, or a combination comprising one or more of the foregoing; all based on the total weight of the respective carbonate compound or diaryl carbonate.

12. The method of any one of the preceding claims, wherein the melt polymerizing comprises consecutively
polymerizing in a first oligomerization unit;
polymerizing in a second oligomerization unit that is in fluid communication with the first oligomerization unit;
polymerizing in a first polymerization unit that is in fluid communication with the second oligomerization unit; and
polymerizing in a second polymerization unit that is in fluid communication with the first polymerization unit to form the polycarbonate;
wherein the one or more overhead streams is withdrawn from one or more of the second oligomerization unit, the first polymerization unit, and the second polymerization unit.

13. A system for purifying an overhead stream from a melt polymerization facility, comprising:
an aromatic alcohol purification unit in fluid communication with the overhead stream from the melt polymerization facility, wherein the aromatic alcohol purification unit is capable of separating a purified aromatic alcohol stream and a purification unit bottom stream;
a diarylcarbonate purification unit that is in fluid communication with the aromatic alcohol purification unit via the purification unit bottom stream, wherein diarylcarbonate purification unit is capable of separating a diaryl carbonate stream and a diaryl carbonate purification unit bottom stream; and
a cracker unit that is in fluid communication with the diarylcarbonate purification unit via the diaryl carbonate purification unit bottom stream and a cracking agent stream, wherein the cracker unit is capable of forming a resultant stream comprising an additional aromatic alcohol.

14. The system of Claim 13, wherein one or both of the aromatic alcohol purification unit and the cracker unit is in fluid communication with a monomer production facility that is capable of forming one or both of a diaryl carbonate and bisphenol.

15. The system of any one of Claims 13-14, further comprising
a separation unit in fluid communication with the cracking unit via the resultant stream, wherein the separation unit is capable of separating the resultant stream into an aromatic alcohol stream, an alkyl carbonate stream, and a bottom stream; preferably wherein the separation unit comprises greater than or equal to two distillation units; or
wherein the cracking unit is capable of separating the resultant stream into an aromatic alcohol stream, an alkyl carbonate stream, and a bottom stream.

## Patentansprüche

1. Ein Verfahren zur Reinigung eines aromatischen Alkohols, das Folgendes umfasst:
das Schmelzpolymerisieren einer Dihydroxyverbindung und einer Carbonatverbindung in Anwesenheit eines Umesterungskatalysators, um den aromatischen Alkohol und ein Polycarbonat in einer Schmelzpolymerisationsanlage zu bilden;
das Entfernen des aromatischen Alkohols in einem Kopfstrom;
das Auftrennen des Kopfstroms, in einer Reinigungseinheit für aromatische Alkohole, in einen gereinigten aromatischen Alkoholstrom, der den aromatischen Alkohol umfasst, und einen unteren Strom der Reinigungseinheit;
das Auftrennen des unteren Stroms der Reinigungseinheit in einer Diarylcarbonat-Reinigungseinheit in einen Diarylcarbonatstrom und einen unteren Strom der Diarylcarbonat-Reinigungseinheit; und
das Kracken einer Verbindung im unteren Strom der Diarylcarbonat-Reinigungseinheit in einer Krackanlage in Anwesenheit eines Krackmittels, um einen Gesamtstrom zu bilden, der zusätzlichen aromatischen Alkohol umfasst.

2. Das Verfahren gemäß Anspruch 1, das weiter eines oder beide von Folgendem umfasst: dem Lenken des gereinigten aromatischen Alkoholstroms an eine Monomerproduktionsanlage, die in Fluidaustausch mit der Reinigungseinheit für aromatische Alkohole steht, und dem Lenken des Trenneinheitsstroms an die Monomerproduktionsanlage, die in Fluidaustausch mit der Krackanlage steht.

3. Das Verfahren gemäß Anspruch 2, das weiter das Bilden eines Diarylcarbonats in der Monomerproduktionsanlage durch Folgendes umfasst:
das Reagieren des aromatischen Alkohols und/oder des zusätzlichen aromatischen Alkohols mit Kohlenmonoxid in Anwesenheit von Sauerstoff und einem Carbonylierungskatalysator, um das Diarylcarbonat zu bilden; oder
das Reagieren des aromatischen Alkohols und/oder des zusätzlichen aromatischen Alkohols mit Phosgen und/oder einem Dialkylcarbonat in Anwesenheit eines zweiten Umesterungskatalysators, um das Diarylcarbonat zu bilden.

4. Das Verfahren gemäß einem beliebigen der Ansprüche 2-3, das weiter das Bilden eines Bisphenols in der Monomerproduktionsanlage durch Folgendes umfasst:
das Reagieren des aromatischen Alkohols und/oder des zusätzlichen aromatischen Alkohols mit einem Keton und/oder einem Aldehyd in Anwesenheit eines Ionenaustauschharzes, um das Bisphenol zu bilden;
wobei der zusätzliche aromatische Alkohol vorzugsweise einen Gehalt von höchstens 10 ppm C₁₋₄-Alkylalkohol und höchstens 10ppm C₁₋₄-Dialkylcarbonat hat.

5. Das Verfahren gemäß einem beliebigen der obigen Ansprüche, wobei der Gesamtstrom weiter Alkylcarbonat umfasst; wobei das Alkylcarbonat vorzugsweise Dimethylcarbonat umfasst, und/oder wobei der Gesamtstrom einen aromatischen Alkoholstrom und einen Alkylcarbonatstrom umfasst und wobei der aromatische Alkoholstrom und der Alkylcarbonatstrom separat entfernt werden; wobei der Gesamtstrom vorzugsweise in einer Trenneinheit in den aromatischen Alkoholstrom, den Alkylcarbonatstrom und einen unteren Strom aufgeteilt wird.

6. Das Verfahren gemäß einem beliebigen der obigen Ansprüche, das Folgendes umfasst: das Entfernen des Diarylcarbonatstroms als Seitenstrom aus der Diarylcarbonatreinigungseinheit und das Entfernen eines oberen Mittelsieder-Stroms aus einem oberen Ende der Diarylcarbonatreinigungseinheit.

7. Das Verfahren gemäß einem beliebigen der obigen Ansprüche, worin der gereinigte aromatische Alkoholstrom mindestens 99 Gewichtsprozent, genauer 99,5 bis 100 Gewichtsprozent, noch genauer 99,7 bis 100 Gewichtsprozent aromatischen Alkohol, basierend auf dem Gesamtgewicht des gereinigten aromatischen Alkoholstroms, umfasst.

8. Das Verfahren gemäß einem beliebigen der obigen Ansprüche, worin das Kracken bei einem oder mehreren von Folgendem stattfindet: einer Temperatur von 100 bis 500°C, genauer 140 bis 250°C, einem Druck von 100 bis 1.000 kPa, genauer 100 bis 250 kPa und einer Verweilzeit von 0,2 bis 20 Stunden oder 0,5 bis 10 Stunden im Krackapparat.

9. Das Verfahren gemäß einem beliebigen der obigen Ansprüche, wobei das Kracken weiter in Anwesenheit eines Katalysators stattfindet und wobei der Katalysator mindestens eines von Folgendem umfasst: einer aromatischen Sulfonsäure, einer Schwefel enthaltenden Aminverbindung, einem Zeolith, Natriumhypophosphit, Aluminiumisopropylat, einem Natriumsalz einer organischen und/oder einer mineralischen Säure und einer Base; wobei der Katalysator vorzugsweise Natriumhydroxid und/oder Dodecylbenzensulfonsäure umfasst; wobei der Katalysator stärker bevorzugt Dodecylbenzensulfonsäure umfasst.

10. Das Verfahren gemäß einem beliebigen der obigen Ansprüche, worin der untere Strom der Diarylcarbonatreinigungseinheit und ein Krackmittel zu der Krackanlage hinzugegeben werden; wobei das Krackmittel vorzugsweise Wasser und/oder einen Alkylalkohol umfasst; wobei der Alkylalkohol vorzugsweise Methanol ist.

11. Das Verfahren gemäß einem beliebigen der obigen Ansprüche, wobei die Carbonatverbindung und/oder das Diarylcarbonat jeweils unabhängig weniger als oder gleich 33 ppb Molybdän, weniger als oder gleich 33ppb Vanadium, weniger als oder gleich 33 ppb Chrom, weniger als oder gleich 75 ppb Titan, weniger als oder gleich 375 ppb Niob, weniger als oder gleich 33 ppb Nickel, weniger als oder gleich 10ppb Zirconium, weniger als oder gleich 10 ppb Eisen oder eine Kombination umfassen, die eines oder mehrere der oben Genannten umfasst; alles basierend auf dem Gesamtgewicht der jeweiligen Carbonatverbindung oder des Diarylcarbonats.

12. Das Verfahren gemäß einem beliebigen der obigen Ansprüche, worin das Schmelzpolymerisieren nacheinander Folgendes umfasst:
das Polymerisieren in einer ersten Oligomerisationseinheit;
das Polymerisieren in einer zweiten Oligomerisationseinheit, die in Fluidaustausch mit der ersten Oligomerisationseinheit steht;
das Polymerisieren in einer ersten Polymerisationseinheit, die in Fluidaustausch mit der zweiten Oligomerisationseinheit steht; und
das Polymerisieren in einer zweiten Polymerisationseinheit, die in Fluidaustausch mit der ersten Polymerisationseinheit steht, um das Polycarbonat zu bilden;
wobei die einen oder mehreren Kopfströme aus einer oder mehreren der zweiten Oligomerisationseinheit, der ersten Polymerisationseinheit und der zweiten Polymerisationseinheit entnommen werden.

13. Ein System zur Reinigung eines Kopfstroms aus einer Schmelzpolymerisationsanlage, das Folgendes umfasst:
eine Reinigungseinheit für aromatische Alkohole, in Fluidaustausch mit dem Kopfstrom von der Schmelzpolymerisationsanlage, wobei die Reinigungseinheit für aromatische Alkohole dazu in der Lage ist, einen gereinigten aromatischen Alkoholstrom und einen unteren Reinigungseinheitsstrom zu trennen;
eine Diarylcarbonatreinigungseinheit, die über den unteren Reinigungseinheitsstrom in Fluidaustausch mit der Reinigungseinheit für aromatische Alkohole steht, wobei die Diarylcarbonatreinigungseinheit in der Lage ist, einen Diarylcarbonatstrom und einen unteren Strom der Diarylcarbonatreinigungseinheit zu trennen; und
eine Krackanlage, die über den unteren Strom der Diarylcarbonatreinigungseinheit und einen Krackmittelstrom in Fluidaustausch mit der Diarylcarbonatreinigungseinheit steht, wobei die Krackanlage in der Lage ist, einen Gesamtstrom zu bilden, der einen zusätzlichen aromatischen Alkohol umfasst.

14. Das System gemäß Anspruch 13, wobei die aromatische Alkoholreinigungseinheit und/oder die Krackanlage in Fluidaustausch mit einer Monomerproduktionsanlage stehen, die in der Lage ist, ein Diarylcarbonat und/oder Bisphenol zu bilden.

15. Das System gemäß einem beliebigen der Ansprüche 13-14, das weiter Folgendes umfasst:
eine Trenneinheit in Fluidaustausch mit der Krackanlage über den Gesamtstrom, wobei die Trenneinheit in der Lage ist, den Gesamtstrom in einen aromatischen Alkoholstrom, einen Alkylcarbonatstrom und einen unteren Strom aufzuteilen, wobei die Trenneinheit vorzugsweise mindestens zwei Destillationseinheiten umfasst; oder
wobei die Krackanlage in der Lage ist, den Gesamtstrom in einen aromatischen Alkoholstrom, einen Alkylcarbonatstrom und einen unteren Strom aufzuteilen.

## Revendications

1. Procédé de purification d'un alcool aromatique comprenant :
la polymérisation en masse fondue d'un composé à fonction dihydroxy et d'un composé à fonction carbonate en présence d'un catalyseur de transestérification pour former l'alcool aromatique et un polycarbonate dans une installation de polymérisation en masse fondue ;
l'extraction de l'alcool aromatique dans un flux de tête ;
la séparation du flux de tête dans une unité de purification d'alcool aromatique en un flux d'alcool aromatique purifié comprenant l'alcool aromatique et un flux de fond d'unité de purification ;
la séparation du flux de fond d'unité de purification dans une unité de purification de carbonate de diaryle en un flux de carbonate de diaryle et un flux de fond d'unité de purification de carbonate de diaryle ; et
le craquage d'un composé dans le flux de fond d'unité de purification de carbonate de diaryle dans une unité de craqueur en présence d'un agent de craquage pour former un flux résultant comprenant de l'alcool aromatique supplémentaire.

2. Procédé selon la revendication 1, comprenant en outre un ou plusieurs du guidage du flux d'alcool aromatique purifié vers une installation de production de monomère qui est en communication fluidique avec l'unité de purification d'alcool aromatique et du guidage du flux d'unité de séparation vers l'installation de production de monomère qui est en communication fluidique avec l'unité de craqueur.

3. Procédé selon la revendication 2, comprenant en outre la formation d'un carbonate de diaryle dans l'installation de production de monomère par
réaction d'un ou plusieurs de l'alcool aromatique et de l'alcool aromatique supplémentaire avec du monoxyde de carbone en présence d'oxygène et d'un catalyseur de carbonylation pour former le carbonate de diaryle ; ou
réaction d'un ou plusieurs de l'alcool aromatique et de l'alcool aromatique supplémentaire avec du phosgène et/ou un carbonate de dialkyle en présence d'un second catalyseur de transestérification pour former le carbonate de diaryle.

4. Procédé selon l'une quelconque des revendications 2 à 3, comprenant en outre la formation d'un bisphénol dans l'installation de production de monomère par
réaction d'un ou plusieurs de l'alcool aromatique et de l'alcool aromatique supplémentaire avec une cétone et/ou un aldéhyde en présence d'une résine échangeuse d'ions pour former le bisphénol ;
de préférence dans lequel l'alcool aromatique supplémentaire a une teneur inférieure ou égale à 10 ppm d'alcool alkylique en C₁₋₄, et inférieure ou égale à 10 ppm de carbonate de dialkyle en C₁₋₄.

5. Procédé selon l'une quelconque des revendications précédentes, dans lequel le flux résultant comprend en outre du carbonate d'alkyle ; de préférence dans lequel le carbonate d'alkyle comprend du carbonate de diméthyle ; et/ou dans lequel le flux résultant comprend un flux d'alcool aromatique et un flux de carbonate d'alkyle, et dans lequel le flux d'alcool aromatique et le flux de carbonate d'alkyle sont extraits séparément ; de préférence dans lequel le flux résultant est séparé dans une unité de séparation en le flux d'alcool aromatique, le flux de carbonate d'alkyle et un flux de fond.

6. Procédé selon l'une quelconque des revendications précédentes, comprenant l'extraction du flux de carbonate de diaryle en tant que flux latéral provenant de l'unité de purification de carbonate de diaryle et l'extraction d'un flux supérieur de produits à point d'ébullition intermédiaire à partir d'un haut de l'unité de purification de carbonate de diaryle.

7. Procédé selon l'une quelconque des revendications précédentes, dans lequel le flux d'alcool aromatique purifié comprend une quantité supérieure ou égale à 99 % en poids, spécifiquement, de 99,5 à 100 % en poids, plus spécifiquement, de 99,7 à 100 % en poids d'alcool aromatique sur la base du poids total du flux d'alcool aromatique purifié.

8. Procédé selon l'une quelconque des revendications précédentes, dans lequel le craquage se produit à un ou plusieurs d'une température de 100 à 500 °C, spécifiquement, de 140 à 250 °C ; d'une pression de 100 à 1 000 kPa, spécifiquement, de 100 à 250 kPa ; et d'un temps de séjour dans le craqueur de 0,2 à 20 heures, ou de 0,5 à 10 heures.

9. Procédé selon l'une quelconque des revendications précédentes, dans lequel le craquage est en outre en présence d'un catalyseur, et dans lequel le catalyseur comprend au moins l'un d'un acide sulfonique aromatique, d'un composé aminé contenant du soufre, d'une zéolite, d'hypophosphite de sodium, d'isopropylate d'aluminium, d'un sel sodique d'un ou plusieurs d'un acide organique et minéral, et d'une base ; de préférence dans lequel le catalyseur comprend au moins l'un de l'hydroxyde de sodium et de l'acide dodécylbenzène sulfonique, plus préférablement dans lequel le catalyseur comprend de l'acide dodécylbenzène sulfonique.

10. Procédé selon l'une quelconque des revendications précédentes, dans lequel le flux de fond d'unité de purification de carbonate de diaryle et un agent de craquage sont ajoutés à l'unité de craquage ; de préférence dans lequel l'agent de craquage comprend au moins l'un de l'eau et d'un alcool alkylique ; de préférence dans lequel l'alcool alkylique est le méthanol.

11. Procédé selon l'une quelconque des revendications précédentes, dans lequel un ou plusieurs du composé à fonction carbonate et du carbonate de diaryle comprennent chacun indépendamment une quantité inférieure ou égale à 33 ppb de molybdène ; inférieure ou égale à 33 ppb de vanadium ; inférieure ou égale à 33 ppb de chrome ; inférieure ou égale à 75 ppb de titane ; inférieure ou égale à 375 ppb de niobium ; inférieure ou égale à 33 ppb de nickel ; inférieure ou égale à 10 ppb de zirconium ; inférieure ou égale à 10 ppb de fer, ou une combinaison comprenant un ou plusieurs des précédents ; tous sur la base du poids total du composé à fonction carbonate ou du carbonate de diaryle respectif.

12. Procédé selon l'une quelconque des revendications précédentes, dans lequel la polymérisation en masse fondue comprend successivement
une polymérisation dans une première unité d'oligomérisation ;
une polymérisation dans une seconde unité d'oligomérisation qui est en communication fluidique avec la première unité d'oligomérisation ;
une polymérisation dans une première unité de polymérisation qui est en communication fluidique avec la seconde unité d'oligomérisation ; et
une polymérisation dans une seconde unité de polymérisation qui est en communication fluidique avec la première unité de polymérisation pour former le polycarbonate ;
dans lequel les un ou plusieurs flux de tête sont extraits d'une ou plusieurs de la seconde unité d'oligomérisation, de la première unité de polymérisation et de la seconde unité de polymérisation.

13. Système de purification d'un flux de tête provenant d'une installation de polymérisation en masse fondue, comprenant :
une unité de purification d'alcool aromatique en communication fluidique avec le flux de tête provenant de l'installation de polymérisation en masse fondue, dans lequel l'unité de purification d'alcool aromatique est capable de séparer un flux d'alcool aromatique purifié et un flux de fond d'unité de purification ;
une unité de purification de diarylcarbonate qui est en communication fluidique avec l'unité de purification d'alcool aromatique par l'intermédiaire du flux de fond d'unité de purification, dans lequel l'unité de purification de diarylcarbonate est capable de séparer un flux de carbonate de diaryle et un flux de fond d'unité de purification de carbonate de diaryle ; et
une unité de craqueur qui est en communication fluidique avec l'unité de purification de diarylcarbonate par l'intermédiaire du flux de fond d'unité de purification de carbonate de diaryle et un flux d'agent de craquage, dans lequel l'unité de craqueur est capable de former un flux résultant comprenant un alcool aromatique supplémentaire.

14. Système selon la revendication 13, dans lequel une ou plusieurs de l'unité de purification d'alcool aromatique et de l'unité de craqueur est en communication fluidique avec une installation de production de monomère qui est capable de former un ou plusieurs d'un carbonate de diaryle et du bisphénol.

15. Système selon l'une quelconque des revendications 13 à 14, comprenant en outre
une unité de séparation en communication fluidique avec l'unité de craquage par l'intermédiaire du flux résultant, dans lequel l'unité de séparation est capable de séparer le flux résultant en un flux d'alcool aromatique, un flux de carbonate d'alkyle et un flux de fond ; de préférence dans lequel l'unité de séparation comprend une quantité supérieure ou égale à deux unités de distillation ; ou
dans lequel l'unité de craquage est capable de séparer le flux résultant en un flux d'alcool aromatique, un flux de carbonate d'alkyle et un flux de fond.
